# EUROPEAN PATENT APPLICATION

(11) **EP 4 342 484 A1**
(43) Date of publication of application: **27.03.2024**
(21) Application number: 22805003.5
(22) Date of filing: 19.05.2022
(51) Int. Cl.: A61K 38/12, A61K 38/16, A61K 45/06, A61P 31/06, A61P 31/04, C07K 14/32, C07K 1/06

(54) **PHARMACEUTICAL COMPOSITION COMPRISING MACROCOSINE COMPOUND AND METHOD FOR PREPARING MACROCOSINE COMPOUND**

(30) Priority: 21.05.2021 KR 20210065438; 18.05.2022 KR 20220061016
(71) Applicant: A&J Science Co.,Ltd., Daegu 41061 (KR)
(72) Inventor: HWANG, Hee-Jong, Daegu 41106 (KR); SON, Young-Jin, Daegu 41104 (KR); KIM, Dahyun, Daegu 41084 (KR); LEE, Jusuk, Daegu 41119 (KR); CLOVIS, Shyaka, Daegu 41063 (KR); CIUFOLINI, Marco, Vancouver, British Columbia V6K 1V5 (CA)
(74) Representative: Potter Clarkson
(86) International application number: PCT/KR2022/007173
(87) International publication number: WO 2022/245150

(57) **Abstract**

The present invention provides a method for preparing a micrococcin compound, and a pharmaceutical composition for the treatment and prevention of infection of a specific strain comprising a micrococcin compound, a solvate thereof, a hydrate thereof, a prodrug thereof, an isomer thereof, or a pharmaceutically acceptable salt thereof as effective component. In addition, the present invention provides an anti-inflammatory composition comprising the micrococcin compound, a solvate thereof, a hydrate thereof, a prodrug thereof, an isomer thereof, or a pharmaceutically acceptable salt thereof as an effective component.

## Description

### [Technical Field]

The present invention relates to a pharmaceutical composition including a specific micrococcin compound and a total synthesis method of a micrococcin compound.

### [Background Art]

Tuberculosis is a major disease in developing countries, which causes about 8 million new infections and kills 3 million people every year, but in recent years, has been emerging as a problem in developed countries also.

Tuberculosis may remain latently infected for a long time depending on a patient's immune status, and it is estimated that 2 billion people which is about 30% of the world's population are latently infected. When a person is infected with tuberculosis, he/she may be asymptomatic for a considerable period of time but may show acute inflammation of the lungs as a common symptom, and as a result, fever and nonproductive cough are caused. When it is not treated, serious complications and death commonly result.

Development of an antituberculosis drug began with streptomycin in the 1940s and more effective antituberculosis drugs were developed, and as a result, the incidence of tuberculosis and deaths resulting therefrom were rapidly decreased.

However, due to the incidence of "multidrug-resistant tuberculosis (MDR-TB)" which is a resistant strain which may not be controlled with existing antibiotics in the 1980s, the number of patients with multidrug-resistant tuberculosis has not decreased, which emerged as a serious health problem.

Another lung disease is a disease caused by nontuberculous mycobacteria (NTM).

NTM is widely distributed in the natural environment, and since an NTM infection occurs in the natural environment, NTM is also referred to as environmental mycobacteria. Most NTM is less pathogenic for humans than tuberculosis bacteria. Therefore, an NTM infectious disease accompanied by symptoms may be associated with local or systemic immune defects. Colonization, contamination of a specimen, or the like as well as an actual NTM infection may cause NTM to be separated from a clinical specimen.

Since the tuberculosis and the diseases caused by nontuberculous mycobacteria are characterized in that they are chronic and difficult to cure due to their resistance, development of therapeutic agents for them is urgently needed.

Meanwhile, thiopeptide is an antibiotic group produced by actinomyces or bacteria and has been studied a lot due to its broad biological profile and outstanding efficacy.

Micrococcin is a thiopeptide natural product, has a structural feature of having a macrocycle composed of 26 atoms, and is classified into micrococcin Pa (MP1) and micrococcin P2 (MP2). Micrococcin P1 is known to have efficacy against various gram-positive bacteria, and shows an antibacterial action with a mechanism of action to bind to a composite formed between L11 protein and 23S rRNA and inhibit protein synthesis.

Micrococcin P1 is a microcyclic peptide antibiotic mainly found on the surface of French Raclette cheese, identified from *Staphylococcus equorum* WS2733, and its total synthesis has been already completed.

Micrococcin is known to exist as P1 and P2 at 7:1 in *Bacillus pumilus,* but it is very difficult to separate micrococcin P2 and its total synthesis has not been reported.

The biological activity of micrococcin P2 purified as mentioned above has been reported, but little has been reported about the rest. Further, the antibacterial effect against tuberculosis bacteria, nontuberculous mycobacteria, and *clostridium* bacteria has not been reported.

### [Disclosure]

### [Technical Problem]

Thus, the inventors of the present invention studied and completed a total synthesis method of a micrococcin compound, and found that the micrococcin compound is surprisingly effective for killing and suppressing specific bacteria, thereby completing the present invention.

An object of the present invention is to provide a total synthesis method of a micrococcin compound.

Another object of the present invention is to provide a pharmaceutical composition including a micrococcin compound, a solvate thereof, a hydrate thereof, a prodrug thereof, an isomer thereof, or a pharmaceutically acceptable salt thereof as an effective component.

Still another object of the present invention is to provide an anti-inflammatory composition including a micrococcin compound, a solvate thereof, a hydrate thereof, a prodrug thereof, an isomer thereof, or a pharmaceutically acceptable salt thereof as an effective component.

### [Technical Solution]

In one general aspect, a pharmaceutical composition for treating or preventing a bacterial infection caused by tuberculosis bacteria or nontuberculous mycobacteria includes: a micrococcin compound which may effectively kill specific bacteria, tuberculosis bacteria and nontuberculous mycobacteria or inhibit their growth, a solvate thereof, a hydrate thereof, a prodrug thereof, an isomer thereof, or a pharmaceutically acceptable salt thereof as an effective component, wherein the micrococcin compound of the present invention is represented by the following Chemical Formula 1:

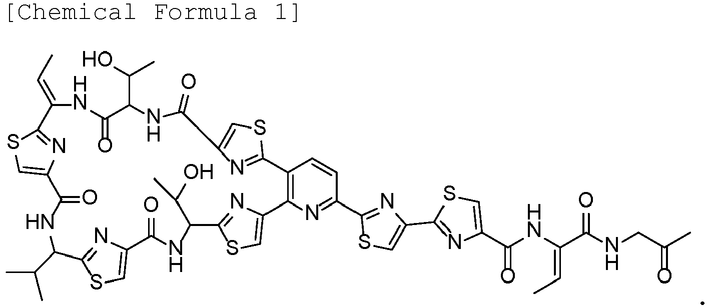

In another general aspect, a pharmaceutical composition for treating and preventing a bacterial infection caused by *Clostridium* (*Clostridium sp.*) bacteria includes: the micrococcin compound represented by Chemical Formula 1, a solvate thereof, a hydrate thereof, a prodrug thereof, an isomer thereof, or a pharmaceutically acceptable salt thereof.

In another general aspect, an anti-inflammatory composition includes the micrococcin compound represented by Chemical Formula 1, a solvate thereof, a hydrate thereof, a prodrug thereof, an isomer thereof, or a pharmaceutically acceptable salt thereof as an effective component.

In still another general aspect, a method for preparing the micrococcin compound represented by Chemical Formula 1.

### [Advantageous Effects]

The pharmaceutical composition of the present invention includes a specific compound, a micrococcin compound as an effective component, thereby having a surprisingly excellent antibiotic effect against specific bacteria, tuberculosis bacteria, nontuberculous mycobacteria, and *clostridium* bacteria and being very effective for treating and preventing infections caused by the bacteria.

Furthermore, the pharmaceutical composition of the present invention also has a very good effect on treatment and prevention of multidrug-resistant or extensive drug-resistant tuberculosis and may effectively treat and prevent tuberculosis.

In addition, the method for preparing a micrococcin compound of the present invention produces a micrococcin compound by total synthesis, unlike a conventional method of obtaining the micrococcin compound by separation from a natural product, thereby preparing a micrococcin compound having excellent purity with a high yield.

Therefore, the method for preparing a micrococcin compound of the present invention may produce the compound by chemical synthesis, not by separation from a natural product, which allows mass production and low-cost commercialization.

In addition, the micrococcin compound according to the present invention shows an anti-inflammatory effect to inhibit production of inflammatory cytokines, and thus, may be used as pharmaceutical, functional food, and cosmetic materials for treating, preventing, and improving inflammation-related diseases.

### [Description of Drawings]

FIG. 1 is results of evaluating efficacy in a clostridium difficile-infected model.
FIG. 2 is evaluation of anti-inflammatory efficacy.

### [Best Mode]

Hereinafter, a pharmaceutical composition of the present invention, including a micrococcin compound, a solvate thereof, a hydrate thereof, a prodrug thereof, an isomer thereof, or a pharmaceutically acceptable salt thereof as an effective component, will be described in detail. Here, technical terms and scientific terms used in the present specification have the general meaning understood by those skilled in the art to which the present invention pertains unless otherwise defined, and description for the known function and configuration which may unnecessarily obscure the gist of the present invention will be omitted in the following description.

The following terms used in the present specification are defined as follows, but they are only illustrative and do not limit the present invention, application, or use.

In the present specification, the term "protecting group" includes an "amino group-protecting group" or a "hydroxyl group-protecting group", but is not limited thereto. The "amino group-protecting group" refers to a protecting group appropriate for preventing a side reaction at a nitrogen position of an amino group. A representative amino group-protecting group includes a formyl group; an acyl group such as an alkanoyl group, for example, an acetyl group, a trichloroacetyl group, or a trifluoroacetyl group; an alkoxycarbonyl group such as a tert-butoxycarbonyl group (boc); an arylmethoxycarbonyl group such as a benzyloxycarbonyl group (Cbz) and a 9-fluorenylmethoxycarbonyl group (Fmoc); an arylmethyl group such as a benzyl group (bn), a triphenylmethyl group (Tr), and a 1,1-bis-(4'-methoxyphenyl)methyl group; a silyl group such as a trimethylsilyl group (TMS) and a tert-butyldimethylsilyl group (TBS), and the like, but is not limited thereto. The "hydroxyl group-protecting group" refers to a protecting group appropriate for inhibiting a hydroxyl group side reaction. A representative hydroxyl group-protecting group includes an alkyl group such as a methyl group, an ethyl group, and a tert-butyl group; an acyl group such as an alkanoyl group (for example, acetyl group); an arylmethyl group such as a benzyl group (Bn), p-methoxybenzyl group (PMB), 9-fluorenylmethyl group (Fm), and a diphenylmethyl group (DPM); a silyl group such as a trimethylsilyl group (TMS) and a tert-butyldimethylsilyl group (TBS), and the like, but is not limited thereto.

The term "halogen" in the present specification refers to fluorine, chlorine, bromine, or iodine.

The term "pharmaceutically acceptable salt" in the present specification includes salts of active compounds prepared from a relatively non-toxic acid and a base depending on a specific substituent found in the compounds mentioned herein. When the compounds of the present invention includes a relatively acidic functionality, base addition salts may be obtained by bringing a neutral form of the compounds into contact with a desired base and a pure or appropriate inert solvent in a sufficient amount. An example of the pharmaceutically acceptable base addition salt includes a sodium, potassium, calcium, ammonium, organic amino, or magnesium salt, or similar salts. When the compounds of the present invention include a relatively basic functionality, acid addition salts may be obtained by bringing a neutral form of the compounds into contact with a desired acid and pure or appropriate inert solvent in a sufficient amount. An example of the pharmaceutically acceptable acid addition salt includes not only salts derived from relatively non-toxic organic acids including acetic acid, propionic acid, isobutylic acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and analogues thereof, but also salts derived from hydrogen chloride, hydrogen bromide, nitric acid, carbonic acid, monohydrogencarbonic acid, phosphoric acid, monohydrophosphoric acid, dihydrophosphoric acid, sulfuric acid, monohydrosulfuric acid, hydrogen iodide, or phosphorus acid, and analogues thereof. In addition, it includes salts of an amino acid such as arginate and analogues thereof, and analogues of an organic acid such as glucuronic acid or galactunoric acid and analogues thereof.

Some specific compounds of the present invention have both basic and acidic functionalities which convert compounds into basic or acidic addition salts.

An "effective amount" used in the present specification refers to an amount of the compound of the present invention which is sufficient to provide therapeutic advantages in treating or managing a bacterial infection.

The "effective amount" also refers to an amount of the compound of the present invention which is sufficient to cause death of bacteria or inhibition of a bacterial outbreak. The "effective amount" also refers to an amount sufficient to treat or prevent a bacterial infection either *in vitro* or *in vivo.*

The term "tuberculosis" used in the present specification includes an infection caused by *Mycobacterium tuberculosis* and *Mycodbacterium bovis;* respiratory tuberculosis, for example, tuberculosis of lungs, larynx, trachea, and bronchi, tuberculosis of intrathoracic lymph nodes, tuberculous pleurisy, early respiratory tuberculosis, and other respiratory tuberculosis; nervous system tuberculosis, for example, tuberculous meningitis, tuberculosis of meninges, tuberculous leptomeningitis, meningeal tuberculoma, and other nervous system tuberculosis; tuberculosis of bones and joints, tuberculosis of genitourinary system, tuberculous peripheral lymphadenopathy, tuberculosis of intestine, peritoneum, mesenteric glands, tuberculosis of skin and percutaneous tissue, tuberculosis of eyes, ears, or adrenal glands, and miliary tuberculosis.

A nontuberculous mycobacterium (NTM) refers to a mycobacterium except a tuberculosis bacterium (M. *Tuberculosis*) and a Hansen's bacillus (*M. Leprae*), and diseases caused by the nontuberculous mycobacteria may include lung diseases, lymphadenitis, skin/soft tissue/bone infection, disseminated diseases, or the like, and additionally, may include cough, chronic fatigue, systemic weakness, difficulty in breathing, breast malaise, hemoptysis, or the like, but are not limited thereto.

"Prevention" used in the present specification includes prevention of recurrence, expansion, or outbreak of a bacterial infection.

"Treatment" used in the present specification includes bacterial killing and inhibition.

"Administration" as applied to the present invention represents bringing a therapeutically effective amount of a compound of Chemical Formula 1 and/or a pharmaceutically acceptable salt thereof or a pharmaceutical composition into contact with a patient in need thereof, preferably an animal, most preferably a mammal, and still more preferably a human.

The "patient in need thereof" refers to a patient in need of treatment of diseases caused by bacterial infection. In an aspect of the present invention, the "patient in need thereof" refers to any patient who may have bacterial infection or be at risk of having a bacterial infection. Preferably, the patient in need thereof refers to an animal, more preferably a mammal, and still more preferably a human.

The "micrococcin compound of the present invention" which is the term used in the present specification has a meaning including not only each compound of Chemical Formulae 1 and 2 but also clathrates, hydrates, solvates, prodrugs, or polymorphs thereof. In addition, the term "micrococcin compound of the present invention" has a meaning also including a pharmaceutically acceptable salt of the compound of the present invention, when a pharmaceutically acceptable salt thereof is not mentioned. In an exemplary embodiment, the micrococcin compound of the present invention may exist as a stereoisomerically pure compound (for example, substantially having no other stereoisomer (e.g., 85% ee or more, 90% ee or more, 95% ee or more, 97% ee or more, or 99% ee or more)). That is, when the compounds of Chemical Formulae 1 and 2 according to the present invention or salts thereof are tautomers and/or stereoisomers (for example, geometrical isomers or conformational isomers), each of their separated isomers and mixtures is also included in the scope of the compound of the present invention. When the compound of the present invention or the salt thereof has asymmetric carbon in the structure, an optical active compound and a racemic compound thereof are also included in the scope of the compound of the present invention. For example, when the compounds of the present invention have a sulfoxide (SOR) structure, they may have chirality. The compound of the present invention includes R and S forms of the isomers, and mixtures of the R and S forms are also included in the scope of the compound of the present invention.

In addition, the compounds of the present invention may exist in any one form of a keto form or an enol form, and the forms are also included in the scope of the compound of the present invention.

When used in the present specification, the term "polymorph" refers to a solid crystal form of the micrococcin compound of the present invention or a composite thereof. A different polymorph of the same compound shows different physical, chemical, and/or spectral properties. Difference in terms of physical properties includes stability (for example, heat or light stability), compressibility and density (important for formulation and production manufacture), and a dissolution rate (which may affect bioavailability), but is not limited thereto. A difference in stability causes chemical reactivity changes (for example, differential oxidation such as faster discoloration when formed of one polymorph than when formed of another polymorph), mechanical properties (for example, transformation of purified fragments stored as a dynamically preferred polymorph into a thermodynamically more stable polymorph), or both of them (purification of one polymorph being more susceptible to decomposition at high humidity). Other physical properties of the polymorphs may affect their processing. For example, one polymorph may be more likely to form a solvate or may be more difficult to be filtered or washed than another polymorph, due to, for example, its form or particle size distribution.

The term "solvate" used in the present specification refers to the micrococcin compound of the present invention or a pharmaceutically acceptable salt thereof, including a stoichiometric amount or non-stoichiometric amount of solvent bound by a non-covalent intermolecular force. Preferred solvents are volatile and non-toxic, and may be administered to a human in a very small amount.

The term "hydrate" used in the present specification refers to the micrococcin compound of the present invention or a pharmaceutically acceptable salt thereof, including a stoichiometric amount or non-stoichiometric amount of water bound by a non-covalent intermolecular force.

The term "clathrate" used in the present specification refers to the micrococcin compound of the present invention in the form of a crystal lattice including a space (for example, a channel) in which guest molecules (for example, solvent or water) are confined, or a salt thereof.

In the case in which any compound (prodrug) is separated in vivo and produces the micrococcin compound of the present invention or the salt thereof, the compound also belongs to the scope of the present invention. In the case in which it is used in the present specification and not mentioned differently, the term "prodrug" refers to the micrococcin compound of the present invention which may be hydrolyzed, be oxidized, and cause a different reaction under biological conditions (in vitro or in vivo) for supplying an active compound, in particular, the micrococcin compound of the present invention. The examples of the prodrug include compounds which are biohydrolyzed to produce the compound of the present invention, including a biohydrolyzable part such as biohydrolyzable amides, biohydrolyzable esters, biohydrolyzable carbamates, biohydrolyzable carbonates, biohydrolyzable ureides, and biohydrolyzable phosphate analogues, but are not limited to the specific embodiments. Preferably, the prodrug of the compound having a carboxyl functional group is a low alkyl ester of a carboxylic acid. The carboxylic ester is usually formed by esterifying a part of the carboxylic acid present in the molecule. The prodrug may be easily prepared using a method well known to a person skilled in the art.

The term "purified" used in the present specification means that a separating body is more than 90% pure, 95% or more pure in an exemplary embodiment, 99% or more pure in another exemplary embodiment, and 99.9% or more pure in still another exemplary embodiment.

A singular form, "one (a/an)" used in the present specification may include a plural form unless otherwise explicitly indicated in the context.

The term "pharmaceutical composition" used in the present invention includes all of a pharmaceutical composition for treating and preventing a bacterial infection caused by tuberculosis bacteria or nontuberculous mycobacteria and a pharmaceutical composition for treating and preventing a bacterial infection caused by *clostridium,* unless otherwise particularly mentioned.

The present invention provides a pharmaceutical composition including a micrococcin compound having a surprising antibacterial effect against specific bacteria, a solvate thereof, a hydrate thereof, a prodrug thereof, an isomer thereof, or a pharmaceutically acceptable salt thereof as an effective component, an embodiment of the present invention provides a pharmaceutical composition for treating and preventing a bacterial infection caused by tuberculosis bacteria or nontuberculous mycobacteria, and still another embodiment of the present invention provides a pharmaceutical composition for treating and preventing a bacterial infection caused by *clostridium* bacteria.

First, an embodiment of the present invention provides a pharmaceutical composition for treating or preventing a bacterial infection caused by tuberculosis bacteria or nontuberculous mycobacteria including: a micrococcin compound which is effective against tuberculosis bacteria or nontuberculous mycobacteria as an antibacterial agent, a solvate thereof, a hydrate thereof, a prodrug thereof, an isomer thereof, or a pharmaceutically acceptable salt thereof as an effective component, wherein the micrococcin compound of the present invention is represented by the following Chemical Formula 1:

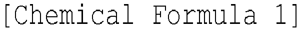

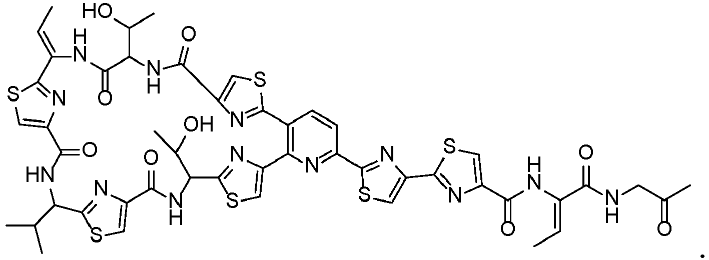

The pharmaceutical composition for treating and preventing a bacterial infection caused by tuberculosis bacteria or nontuberculous mycobacteria of the present invention includes the micrococcin compound represented by Chemical Formula 1, thereby having a very good antibacterial effect against tuberculosis bacteria or nontuberculous mycobacteria in addition to the antibacterial effect against conventionally known bacteria.

Preferably, the micrococcin compound according to an exemplary embodiment may be represented by the following Chemical Formula 2:

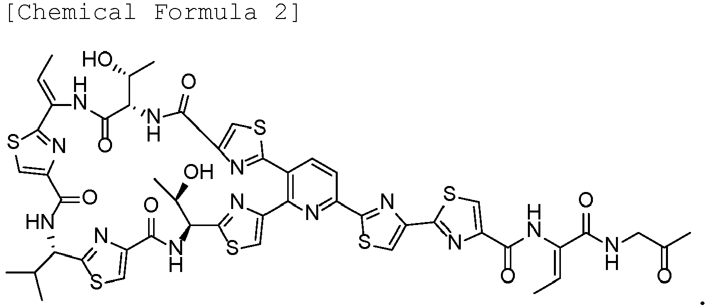

In an exemplary embodiment, the bacterial infection may be tuberculosis or a symptom caused by nontuberculous mycobacteria.

The tuberculosis according to an exemplary embodiment may be an infection caused by *Mycobacterium tuberculosis* or *Mycobacterium bovis,* preferably an infection caused by *Mycobacterium tuberculosis.*

Specifically, tuberculosis according to an exemplary embodiment may include respiratory tuberculosis, for example, tuberculosis of lungs, larynx, trachea, and bronchi, tuberculosis of intrathoracic lymph nodes, tuberculous pleurisy, early respiratory tuberculosis, and other respiratory tuberculosis; nervous system tuberculosis, for example, tuberculous meningitis, tuberculosis of meninges, tuberculous leptomeningitis, meningeal tuberculoma, and other nervous system tuberculosis; tuberculosis of bones and joints, tuberculosis of genitourinary system, tuberculous peripheral lymphadenopathy, tuberculosis of intestine, peritoneum, mesenteric glands, tuberculosis of skin and percutaneous tissue, tuberculosis of eyes, ears, or adrenal glands, and miliary tuberculosis, and the symptoms related thereto may be cough, chest pain, fever, chills, decreased appetite, weight loss, inflammation, or a combination thereof.

The tuberculosis according to an exemplary embodiment may be susceptible tuberculosis, multidrug-resistant (MDR) tuberculosis, or extensive drug-resistant (XDR) tuberculosis. As a specific example, the multidrug-resistant tuberculosis may be tuberculosis in which tuberculosis bacteria are not killed by administering an antibiotic, more specifically, one or more antituberculosis drugs selected from isoniazid, rifampicin, levofloxacin, ofloxacin, moxifloxacin, kanamycin, amikacin, and capreomycin, and as an example, tuberculosis in which tuberculosis bacteria are not killed by administering isoniazid and rifampicin to a human body, and extensive drug-resistant tuberculosis may be tuberculosis resistant to both a quinolone family and an injection as well as isoniazid and rifampicin. The susceptible tuberculosis refers to tuberculosis on which a therapeutic effect of an antibiotic may be exerted as intended, as an opposite concept of resistance to antibiotics induced by a use of the antibiotic.

The symptom caused by nontuberculous mycobacteria according to an exemplary embodiment may be lung diseases, lymphadenitis, skin/soft tissue/bone infection, or disseminated disease, and additionally, may include cough, chronic fatigue, systemic weakness, difficulty in breathing, breast malaise, hemoptysis, or the like, but are not limited thereto.

The nontuberculous mycobacteria according to an exemplary embodiment may be selected from *Mycobacterium avium, Mycobacterium intracellulare, Mycobacterium abscessus, Mycobacterium kansasii, Mycobacterium fortuitum, Mycobacterium gordonae, Mycobacterium osloensis, Mycobacterium phlei, Mycobacterium smegmatis, Mycobacterium terrae, Mycobacterium chelonae, Mycobacterium mucogenicum, Mycobacterium peregrinum, Mycobacterium simiae, Mycobacterium wolinskyi,* or a combination thereof, and more specifically, may be *Mycobacterium avium, Mycobacterium intracellulare, Mycobacterium abscessus, Mycobacterium kansasii, Mycobacterium fortuitum,* or a combination thereof.

The pharmaceutical composition for treating and preventing a bacterial infection caused by tuberculosis bacteria or nontuberculous mycobacteria according to an exemplary embodiment may further include an antibiotic, the antibiotic may be an antituberculosis drug, and the antituberculosis drug may be isoniazid, rifampicin, ethambutol, SQ-109, pyrazinamide, streptomycin, kanamycin, capreomycin, ethionamide, prothionamide, enviomycin, para-aminosalicylic acid, cycloserine, amikacin, levofloxacin, moxifloxacin, Gatifloxacin, ofloxacin, terizidone, thionamide, ethionamide, protionamide, clofazimine, linezolid, amoxicillin, clavulanate, thioacetazone, imipenem, cilastatin, clarithromycin, bedaquiline, delamanid, lmipenem, cilastatin, meropenem, or a combination thereof.

Another embodiment of the present invention provides a pharmaceutical composition for treating and preventing a bacterial infection caused by *clostridium* bacteria including the micrococcin compound of Chemical Formula 1, a solvate thereof, a hydrate thereof, a prodrug thereof, an isomer thereof, or a pharmaceutically acceptable salt thereof as an effective component.

According to an exemplary embodiment, the micrococcin compound of Chemical Formula 1 has an excellent antibacterial effect against *clostridium* bacteria as well as tuberculosis bacteria or nontuberculous mycobacteria.

Preferably, the micrococcin compound according to an exemplary embodiment may be represented by Chemical Formula 2.

In an exemplary embodiment, the bacterial infection caused by *clostridium* bacteria may be colitis, diarrhea, pseudomembranous colitis, gangrenous enteritis, infections of skin and soft tissue, food poisoning, or a combination thereof.

The *clostridium* bacteria according to an exemplary embodiment are not limited, but may be *Clostridium difficile, Clostridium perfringens, Clostridium cadaveris, Clostridium innocuum, Clostridium tetani, Clostridium bifermentans, Clostridium histolyticum, Clostridium clostridioforme, Clostridium subterminale, Clostridium ramosum, Clostridium septicum,* a combination thereof, and preferably, *Clostridium difficile, Clostridium perfringens,* or a combination thereof.

In general, when a *Clostridium difficile* infection (CDI) is treated with an antibiotic, in particular, vancomycin, vancomycin-resistant enterococci (VRE) are increased to cause a problem, but the pharmaceutical composition including the micrococcin compound represented by Chemical Formula 1, the solvate thereof, the hydrate thereof, the prodrug thereof, the isomer thereof, or the pharmaceutically acceptable salt thereof as an effective component allows an effective treatment without increasing the concentration of vancomycin-resistant enterococci (VRE).

Therefore, the pharmaceutical composition for treating and preventing a bacterial infection caused by *clostridium* bacteria including the micrococcin compound of Chemical Formula 1, the solvate thereof, the hydrate thereof, the prodrug thereof, the isomer thereof, or the pharmaceutically acceptable salt thereof as an effective component is very effective for treating a vancomycin-resistant *Clostridium difficile* infection.

The pharmaceutical composition according to an exemplary embodiment may further include one or two or more adjuvants selected from vancomycin, metronidazole, fidaxomicin, ridinilazole, actoxumab, bezlotoxumab, a probiotic, a prebiotic, intravenous immunoglubulin, and an antidiarrheal.

The pharmaceutical composition according to an exemplary embodiment may include a pharmaceutical composition for treating and preventing a bacterial infection caused by tuberculosis bacteria or nontuberculous mycobacteria or a pharmaceutical composition for treating and preventing a bacterial infection caused by *clostridium* bacteria.

In the pharmaceutical composition according to an exemplary embodiment, the micrococcin compound, the solvate thereof, the hydrate thereof, the prodrug thereof, the isomer thereof, or the pharmaceutically acceptable salt thereof may be included at 0.001 to 10 wt% with respect to the total weight of the pharmaceutical composition.

The pharmaceutical composition according to an exemplary embodiment may be administered to a subject before or after outbreak of a bacterial infection. In addition, multiple divided dosages and also a staggered dosage may be administered daily or sequentially, or a dose may be continuously infused or bolus-infused. Furthermore, the dosage of the micrococcin compound of the present invention or the pharmaceutical composition including the same may be increased or decreased proportionally when indicated as a requirement in therapeutic or preventive situations.

In addition, the pharmaceutical composition of the present invention may be used in a pharmaceutical preparation for treating a disease caused by a specific bacterial infection, and the pharmaceutical composition of the present invention includes a preparation appropriate for being administered to a mammal (for example, human).

The pharmaceutical composition according to an exemplary embodiment essentially includes the micrococcin compound of the present invention, a hydrate thereof, a solvate thereof, an isomer thereof, a prodrug thereof, or a pharmaceutically acceptable salt thereof, thereby having high antibacterial activity against tuberculosis bacteria, nontuberculous mycobacteria, or *clostridium* bacteria.

Preferably, the micrococcin compound of the present invention included in the pharmaceutical composition according to an exemplary embodiment of the present invention may be included at 0.001 to 10 wt%, preferably 0.005 to 10 wt%, and more preferably 0.1 to 5 wt% with respect to the total weight of the pharmaceutical composition.

Preferably, the pharmaceutical composition according to an exemplary embodiment of the present invention may further include a pharmaceutically acceptable carrier.

In a specific example, the pharmaceutically acceptable carrier is a material recognized by a person skilled in the art, and includes a pharmaceutically acceptable material, composition, or vehicle appropriate for being administered to a mammal.

The carrier includes a liquid or solid filler, diluent, excipient, solvent, or encapsulated material related to conveyance or transportation of a subject agonist from one organ or body part to another organ or body part. Each carrier should be acceptable in the sense of being compatible with other components of the preparation, and should not be harmful to patients. Some examples of the material which may act as the pharmaceutically acceptable carrier include sugars, for example, lactose, glucose, and sucrose; starches, for example, corn starch and potato starch; cellulose and its derivatives, for example, sodium carboxymethyl cellulose, ethyl cellulose, and cellulose acetate; pulverized tragacanth; malt; gelatin; talc; excipients, for example, cocoa butter and wax for suppositories; oil, for example, peanut oil, cottonseed oil, safflower oil, sesame oil, olive oil, corn oil, and soybean oil; glycol, for example, propylene glycol; polyol, for example, glycerin, sorbitol, mannitol, and polyethylene glycol; esters, for example, ethyl oleate and ethyl laurate; agar; buffers, for example, magnesium hydroxide and aluminum hydroxide; alginic acid; pyrogen-free water; an isotonic saline solution; a linger solution; ethyl alcohol; a phosphate buffer solution; and other commercially available non-toxic material used in pharmaceutical preparations.

In addition, the pharmaceutical composition of the present invention may include a wetting agent, an emulsifier, and a lubricant (for example, sodium lauryl sulfate and magnesium stearate), and also a colorant, a release agent, a coating agent, a sweetener, a flavor, a fragrance, a preservative, and an antioxidant.

An example of the pharmaceutically acceptable antioxidant includes water-soluble antioxidants, for example, ascorbic acid, cysteine hydrochloride, sodium hydrogen sulfate, sodium metabisulfite, sodium sulfite, and the like; fat-soluble antioxidants, for example, ascorbyl palmitate, butylated hydroxyanisole (BHA), butylated hydroxytoluene (BHT), lecithin, propyl galate, α-tocopherol, and the like; and metal chealating agent, for example, citric acid, ethyloenediamine tetraacetic acid (EDTA), sorbitol, tartaric acid, phosphoric acid, and the like.

The pharmaceutical composition of the present invention includes those appropriate for oral, inhalational, intranasal, topical, buccal, sublingual, rectal, intravaginal, and/or parenteral administration, may be conveniently provided as a unit dosage form, and may be prepared by any method well known in the pharmaceutical field. An amount of an active component to produce a single dosage form in combination with a carrier material generally corresponds to an amount of a compound which causes a therapeutic effect.

The method for preparing a pharmaceutical composition (or preparation) according to an exemplary embodiment of the present invention includes combining the micrococcin compound of the present invention with a carrier and optionally one or more auxiliary components. In general, the preparation is manufactured by homogeneously and densely combining the micrococcin compound of the present invention with a liquid carrier, a pulverized solid carrier, of both of them, and then, if necessary, molding the product.

The preparation of the present invention appropriate for oral administration may be in the form of capsules, cachets, pills, tablets, lozenges (scented base, usually using sucrose and acacia or tragacanth), powders, or granules, or may be a solution or suspension in an aqueous or non-aqueous liquid, an oil-in-water or water-in-oil liquid emulsion, elixir or syrup, pastilles (inert base, for example, using gelatin and glycerin or sucrose and acacia), and/or mouth cavity cleaners, and the like, and each of them includes a predetermined amount of the micrococcin compound of the present invention as an active component. The micrococcin compound of the present invention may also be administered as a bolus, an electuary, or a paste.

In the solid dosage form of the present invention for oral administration (capsules, tablets, pills, dragees, powders, granules, and the like), the micrococcin compound as the active component is mixed with any one of one or more pharmaceutically acceptable carriers, for example, sodium citrate or dibasic calcium phosphate, and/or a filler or an extender, as an example, starch, lactose, sucrose, glucose, mannitol, and/or silicic acid; a binder, as an example, carboxymethyl cellulose, algniate, gelatin, polyvinyl pyrrolidone, sucrose, and/or acacia; a desiccant, as an example, glycerol; a disintegrant, as an example, agar-agar, calcium carbonate, potato, or tapioca starch, algninc acid, specific silicate, and sodium carbonate; a solution retardant, as an example, paraffin; an absorption accelerator, as an example, a quaternary ammonium compound; a wetting agent, as an example, cetyl alcohol and glycerol monostearate; an absorbent, as an example, kaolin and bentonite clay; a lubricant, as an example, talc, calcium stearate, magnesium stearate, solid polyethylene glycol, sodium lauryl sulfate, and a mixture thereof; and a colorant.

In the case of capsules, tablets, and pills, the pharmaceutical composition may also include a buffer. A solid composition of a similar type may also be used as a filler in a soft- and hard-filled gelatin capsule using lactose or milk sugar, and an excipient such as high molecular weight polyethylene glycol.

The tablet may be manufactured by compression or molding with any one or more auxiliary components, and the compressed tablet may be prepared using a binder (for example, gelatin or hydroxypropyl cellulose), a lubricant, an inert diluent, a preservative, a disintegrant (for example, sodium starch glycolate or crosslinked sodium carboxymethyl cellulose), a surfactant, or a dispersing agent. The molded tablet may be manufactured by molding a mixture of a pulverized compound which is wetted by an inert liquid diluent in an appropriate machine.

The tablets, and the dragees, the pills, and the granules which are another solid dosage forms of the pharmaceutical composition of the present invention may be optionally notched or manufactured to have a coating and a shell such as an enteric coating and other coatings well known in the pharmaceutical formulation field. These may also be formulated to provide a slow or controlled release of an active component therein by using, for example, hydroxypropylmetyl cellulose at various ratios, thereby providing a desired release profile, other polymer matrix, liposomes, and/or microspheres. These may be sterilized by, for example, filtration through a bacteria retaining filter or incorporating a sterilizing agent in a sterile solid composition form which may be dissolved in sterile water or some other sterile injectable medium immediately before use.

The pharmaceutical composition of the present invention may optionally include an opacifying agent, and may be a composition which may be an active component(s) alone or preferentially in a specific area of a gastrointestinal tract, optionally in a delayed manner. An example of a usable embedding composition includes a polymerizable material and wax. The active component may also be present in a microcapsulated form with one or more excipients described above, if appropriate.

A liquid dosage form for oral administration of the micrococcin compound of the present invention or the pharmaceutical composition includes pharmaceutically acceptable emulsions, microemulsions, solutions, suspensions, syrups, and elixirs. The liquid dosage form may include inert diluents commonly used in the art, for example, water or other solvents, solubilizers, and emulsifiers, for example, ethyl alcohol, isopropyl alcohol, ethyl carbonate, ethyl acetate, benzyl alcohol, benzyl benzoate, propylene glycol, 1,3-butylene glycol, oils (in particular, cottonseed oil, peanut oil, corn oil, germination oil, olive oil, castor oil, and sesame oil), glycerol, tetrahydrofuryl alcohol, Labrasol, polyethylene glycol, and fatty acid esters of sorbitan, and mixtures thereof, in addition to the active component.

When the pharmaceutical composition of the present invention is an oral composition, it may also include adjuvants, for example, a wetting agent, an emulsifier, a suspending agent, a sweetening, a flavoring, a colorant, a fragrance, and a preservative, in addition to the inert diluent. A suspension may include, for example, a suspending agent such as ethoxylated isostearyl alcohol, polyoxyethylene sorbitol, and sorbitan ester, mycrocrystalline cellulose, aluminum methahydroxide, bentonite, agar-agar, tragacanth, and a mixture thereof, in addition to the compound of the present invention.

The pharmaceutical composition (preparation) of the present invention for rectal or intravaginal administration may be provided as a suppository, may be prepared by mixing one or more micrococcin compounds of the present invention with, for example, one or more appropriate non-irritating excipients or carriers including cocoa butter, polyethylene glycol, suppository wax, or salicylate, and is solid at room temperature but liquid at body temperature, and thus, will be melted in the rectum or vaginal cavity to release the active compound.

The pharmaceutical composition (preparation) of the present invention appropriate for intravaginal administration also includes pessaries, tampons, creams, gels, pastes, foam, or spray preparations including a carrier which is known to be appropriate in the art.

A dosage form for topical or transdermal administration of the micrococcin compound of the present invention or the pharmaceutical composition includes powder, spray, ointment, paste, cream, lotion, gel, solution, patch, and inhalant. The active compound may be mixed with a carrier which is pharmaceutically acceptable under sterile conditions and any preservative, buffer, or propellant which may be required.

The ointment, the paste, the cream, and the gel may include excipients such as animal and vegetable fats, oils, waxes, paraffin, starch, tragacanth, cellulose derivatives, polyethylene glycol, silicone, bentonite, silicic acid, talc, zinc oxide, or mixtures thereof, in addition to the micrococcin compound of the present invention.

The powder and the spray may include excipients such as lactose, talc, silicic acid, aluminum hydroxide, calcium silicate, polyamide powder, or mixtures thereof, in addition to the micrococcin compound of the present invention. The spray may further include a common propellant, for example, chlorofluorohydrocarbon and volatile unsubstituted hydrocarbon, for example, butane and propane.

The transdermal patch has an additional advantage of providing the controlled delivery of the micrococcin compound of the present invention to a body. The dosage form as such may be manufactured by dissolving or dispersing the micrococcin compound of the present invention in an appropriate medium.

In order to increase the flow rate of the micrococcin compound of the present invention through the skin, an absorption enhancer may also be used. The flow speed may be controlled by provision of a speed-controlled film or dispersion of an active compound in a polymer matrix or gel.

An ophthalmic preparation, an eye ointment, a powder, a solution, and the like are also included in the scope of the right of the present invention.

The pharmaceutical composition of the present invention appropriate for parenteral administration includes one or more micrococcin compounds of the present invention in combination with one or more pharmaceutically acceptable sterile isotonic aqueous solutions or non-aqueous solutions, dispersions, suspensions, or emulsions, or sterile powders which may be reconstituted in a sterile injectable solution or dispersion immediately before use, and may include a solute, a suspending agent, or a thickening agent which makes an antioxidant, a buffer, a bacteriostatic agent, or a preparation isotonic with the blood of a recipient.

An example of an appropriate aqueous and non-aqueous carrier which may be used in the pharmaceutical composition of the present invention includes water, ethanol, Labrasol, polyol (for example, glycerol, propylene glycol, polyethylene glycol, and the like), an appropriate mixture thereof, vegetable oil (for example, olive oil), and injectable organic ester (for example, ethyl oleate). Appropriate flowability may be maintained by, for example, the use of a coating material such as lecithin, the maintenance of the required particle size in the case of a dispersion, and the use of a surfactant. Furthermore, the pharmaceutical composition of the present invention may also include adjuvants such as a preservative, a wetting agent, an emulsifier, and a dispersing agent. It includes various antibiotics and antifungal agents, for example, paraben, chlorobutanol, phenol sorbic acid, and the like, thereby preventing microbial activity. In addition, it may be preferred that an isotonic agent such as sugars and sodium chloride is included in the composition.

In addition, the injectable pharmaceutical composition (preparation) of the present invention includes an agent to delay absorption, for example, aluminum monostearate and gelatin, thereby extending absorption.

A preparation including the pharmaceutical composition of the present invention may be provided orally, parenterally, topically, or rectally. This is provided in a form appropriate for each administrated route, of course. For example, this is administered by a tablet or capsule form by injection or inhalation, or injection, insufflation, or inhalation of eye drop, ointment, suppository, and the like; topically by lotion or ointment; and rectally by a suppository. Preferably, it may be administered orally and/or intravenously, or by inhalation (as an example, liposome, nanoformulation, and the like).

The pharmaceutical composition of the present invention may be administered by various methods acceptable in the art, and as an example, may be administered by administration other than intestinal and topical administration, usually parenteral administration which usually means an administration manner by injection, or parenterally. That is, intravenous, intramuscular, intraarterial, intradural, intracapsular, intraorbital, intracardiac, intradermal, intraperitoneal, intratracheal, subcutaneous, subepidermal, intraarticular, subcapsular, subarachnoid, intraspinal, and intrasternal injections and infusions are included, but the present invention is not limited thereto. Likewise, any number of administrations and any amount of administration are possible as long as they are in various levels acceptable in the art.

In addition, the pharmaceutical composition (preparation) of the present invention may be used in combination of other agonists, for example, an additional antibiotic which is the compound of the present invention or is not the compound of the present invention, for treating a bacterial infection of a subject.

The antibiotic includes antibiotics, biocides, antimicrobials, and bacteriostatic agents, and any antibiotics, biocides, antimicrobials, and bacteriostatic agents are possible as long as they are known types.

In addition, the pharmaceutical composition of the present invention may be formulated so that it may be administered separately from any additional agonist, and may be formulated together so that they are administered all at once. For example, the pharmaceutical composition of the present invention is formulated into one dosage form, and then may be formulated into another dosage form with an additional agonist. Any separate dosage form may be administered simultaneously or at different times.

As another method, the pharmaceutical composition of the present invention may include an additional agonist described in the present invention, and each component may be provided as a separate composition, a composite composition, or a single composition.

Another embodiment of the present invention provides an anti-inflammatory composition including the micrococcin compound represented by Chemical Formula 1, a solvate thereof, a hydrate thereof, a prodrug thereof, an isomer thereof, or a salt thereof as an effective component.

Preferably, the micrococcin compound according to an exemplary embodiment may be represented by Chemical Formula 2.

The term "anti-inflammation" refers to prevention or treatment of inflammation. Herein, the inflammation refers to a disease caused by invasion of external infectious agents (bacteria, mold, viruses, various types of allergens, and the like), and the disease includes atopic dermatitis, allergic dermatitis, inflammatory bowel disease, gastric ulcer, asthma, or the like, but is not particularly limited thereto.

In the above, the salt of the micrococcin compound may be in the form of a pharmaceutically, cosmetically, or sitologically acceptable salt. As the salt, an acid addition salt formed by a pharmaceutically, cosmetically, or sitologically acceptable free acid or a metal salt formed by a base may be useful, and the salt may be as described above.

The anti-inflammatory composition of the present invention includes the micrococcin compound represented by Chemical Formula 1, thereby showing an anti-inflammatory effect improved by inflammatory cytokine inhibitory activity such as IL-6 and IL-1β.

In addition, the present invention provides a method for preparing the micrococcin compound of the present invention, and the method for preparing the micrococcin compound of the present invention includes:
subjecting a compound of the following Chemical Formula 3 to a boronization reaction to prepare a compound of the following Chemical Formula 4; and
subjecting the compound of the following Chemical Formula 4 to a coupling reaction with a compound of the following Chemical Formula 5 to prepare a micrococcin compound represented by the following Chemical Formula 1:

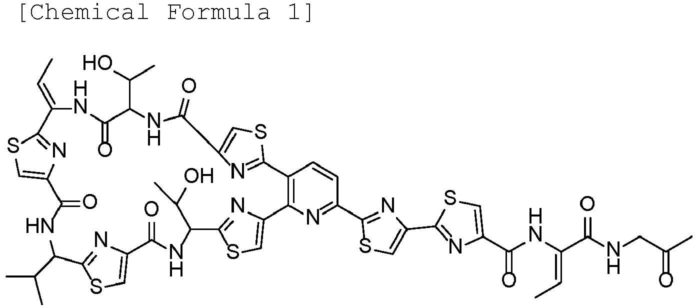

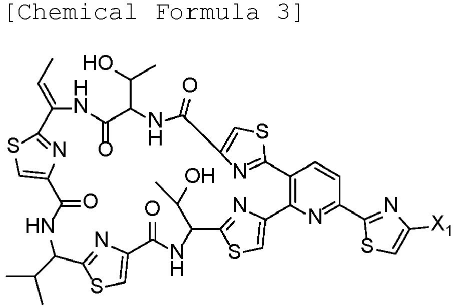

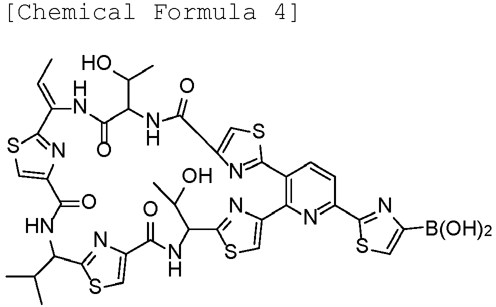

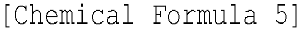

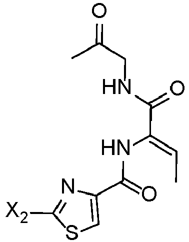

   wherein
   X₁ and X₂ are independently of each other a halogen.

The method for preparing a micrococcin compound of Chemical Formula 1 according to the present invention uses the compound of Chemical Formula 3 which is a compound to which a halogen functional group is introduced, thereby easily substituting a halogen group of Chemical Formula 3 with a boronic acid functional group and subsequently performing an aryl-aryl coupling reaction to prepare the micrococcin compound of Chemical Formula 1 at a high yield in mild conditions.

As the aryl-aryl coupling, as an example, Moto coupling, Suzuki coupling, Negishi coupling, Stille coupling, Sonogashira coupling, Heck coupling, or Buchwald coupling may be appropriate, but a Suzuki coupling reaction is particularly preferred.

The boronization reaction according to an exemplary embodiment may proceed using a boronic acid precursor, the boronic acid precursor may be any organic boronic acid compound which is a compound to which a boronic acid or a boronic acid derivative group may be introduced by reacting with Chemical Formula 3 in the presence of a catalyst, and as an example, may be bis(pinacolato)diboron(B₂pin₂)), bis(catecholato)diborane(B₂Cat₂), or the like.

The aryl-aryl coupling according to an exemplary embodiment may be performed preferably by the Suzuki coupling reaction under a transition metal catalyst.

As the transition metal catalyst, a Pd(0) composite or a Pd(II) salt may be used. A preferred Pd(0) composite is a Pd(0) composite containing one or more phosphine-based ligands, and the phosphine-based ligand may be triphenylphosphine (Ph₃P), tris(ortho-tolyl)phosphine (Pd(o-Tol)₄), Xphos (2-dicyclohexylphosphino-2',4',6'-triisopropylbiphenyl), Johnphos (2-biphenyl)di-tert-butylphosphine), and the like. Preferably, as the Pd(0) composite, Pd(Ph₃P)₄ may be used. A preferred Pd(II) salt includes palladium acetate, that is, Pd(OAc)₂, but is not limited thereto. Another preferred Pd(II) salt may be Pd(dtbpf)Cl₂.

The Suzuki coupling may be performed in the presence of sodium carbonate, potassium phosphate, or organic base, for example, tetraethylammonium carbonate.

Preferably, the compound of Chemical Formula 3 according to an exemplary embodiment may be prepared by including: reacting a compound of the following Chemical Formula 3-1 with a compound of the following Chemical Formula 3-2 to prepare a compound of the following Chemical Formula 3-3; and subjecting the compound of Chemical Formula 3-3 to deprotection and intracyclic reactions, thereby preparing the compound of Chemical Formula 3:

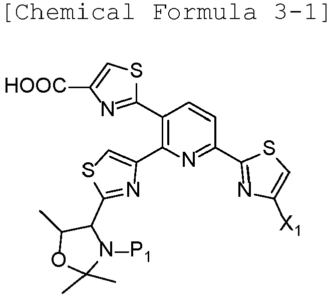

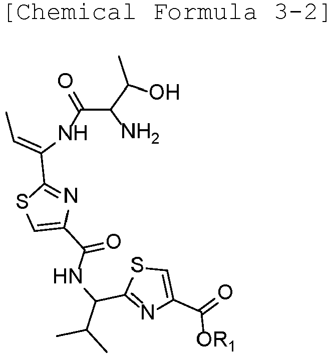

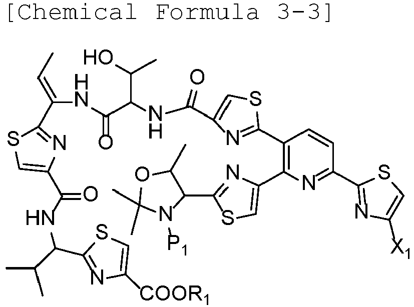

wherein
R₁ is independently of each other C1-C5 alkyl;
P₁ is a protection group; and
X₁ is a halogen.

The deprotection according to an exemplary embodiment is to release a protection group, the protection group may be any protection group which may be used by a person skilled in the art of the present invention, and the protection group of the present invention is a hydroxyl protection group or an amine protection group. The intracyclic reaction according to an exemplary embodiment of the present invention is also possible by a method used in the organic synthesis field, of course.

In a specific example, P₁ may be one amine protection group selected from the group consisting of t-butyloxycarbonyl (Boc), carbobenzyloxy (Cbz), 9-fluorenylmethyloxycarbonyl (Fmoc), acetyl (Ac), benzoyl (Bz), benzyl (Bn), p-methoxybenzyl (PMB), 3,4-dimethoxybenzyl (DMPM), p-methoxyphenyl (PMP), tosyl (Ts), 2,2,2-trichloroethoxycarbonyl (Troc), 2-trimethylsilylethoxycarbonyl (Teoc), and aryloxycarbonyl (Alloc).

The compound of Chemical Formula 3-1 according to an exemplary embodiment may be prepared by including: reacting a compound of the following Chemical Formula 2-1 and ammonium acetate to prepare a compound of the following Chemical Formula 2-2; reacting the compound of Chemical Formula 2-2 with a compound of the following Chemical Formula 2-3 to prepare a compound of the following Chemical Formula 2-4; and reacting the compound of Chemical Formula 2-4 with cysteine to prepare the compound of Chemical Formula 3-1. 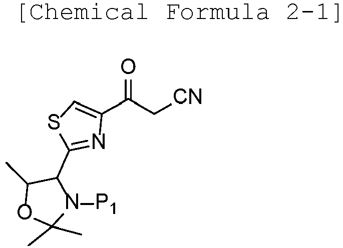 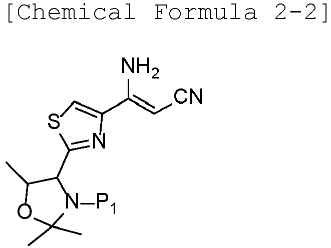 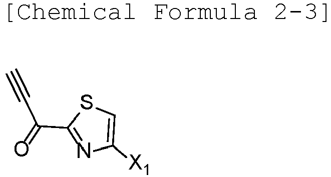 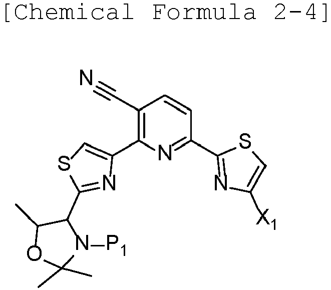 wherein
P₁ is a protection group; and
X₁ is a halogen.

A reaction time in the method for preparing a micrococcin compound of Chemical Formula 1 according to an exemplary embodiment may vary depending on reaction materials, the type of solvent, and the amount of solvent, and as an example, the reaction is completed after confirming that the starting material is completely consumed by TLC and the like. When the reaction is completed, the solvent is distilled under reduced pressure, or a target may be separated and purified by a common method such as tube chromatography after concentration under reduced pressure.

Hereinafter, the method for preparing a micrococcin compound of the present invention will be described with specific examples, but the present invention is not defined by the specific examples.

### [Preparation Example 1] Preparation of Compound A1

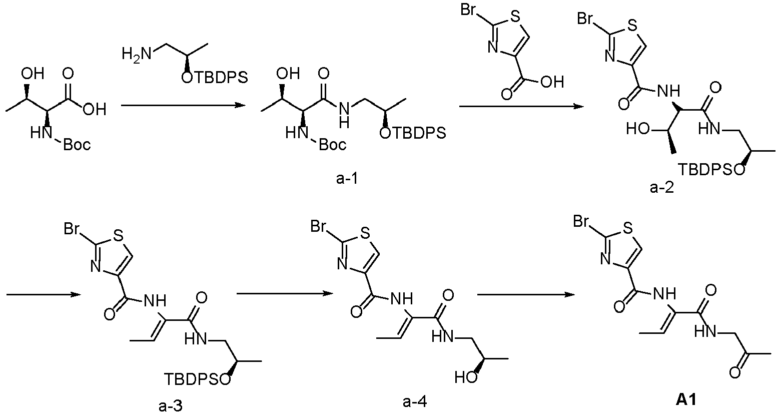

### Step 1: Preparation of Compound a-1

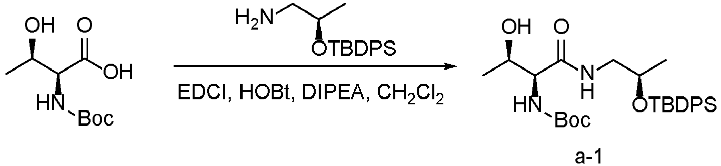

N-Boc-L-threonine (2 g, 9.1 mmol) and (*R*)-2-((*tert-*butyldiphenylsilyl)oxy)propane-1-amine (3.45 g, 11 mmol, 1.2 eq) were dissolved in dichloromethane (40 mL), 1-ethyl-3-(3-dimethylaminopropyl)carbodiimide (EDCI, 2.1 g, 11 mmol, 1.2 eq), hydroxybenzotriazole (HOBt, 1.48 g, 11 mmol, 1.2 eq), and diisopropylethylamine (DIPEA, 3.17 ml, 18.2 mmol, 2 eq) were added dropwise, and stirring was performed at room temperature for 2 hours. Dilution with dichloromethane and washing with water were performed. The organic solvent was extracted, dehydration with anhydrous sodium sulfate was performed, and separation was performed using column chromatography with a 20%-40% ethyl acetate/hexane solution to obtain white solid Compound a-1 (2.80 g, 60%).

¹H NMR (600 MHz, CDCl₃): δ 7.69 (m, 4H), 7.49 - 7.44 (m, 2H), 7.43 - 7.39 (m, 4H), 6.89 (brs, 1H), 5.47 (d, *J* = 7.9 Hz, 1H), 4.34 (qd, *J* = 6.4, 2.0 Hz, 1H), 4.05 - 3.97 (m, 2H), 3.34 - 3.30 (m, 1H), 3.24 (dt, *J* = 13.5, 4.5 Hz, 1H), 2.87 (brs, 1H), 1.48 (s, 9H), 1.18 (d, *J* = 6.4 Hz, 3H), 1.10 (s, 9H), 1.06 (d, J = 6.2 Hz, 3H); LCMS : 515.2 (M+H⁺) for C₂₈H₄₃N₂O₅Si.

### Step 2: Preparation of Compound a-2

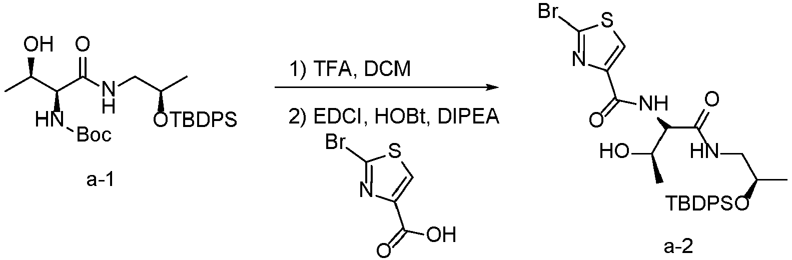

Compound a-1 (4.50 g, 8.8 mmol, 1.1 eq) was dissolved in dichloromethane (40 mL)/ trifluoroacetic acid (40 mL), stirring was performed at room temperature for 1 hour, and concentration under reduced pressure was performed. The obtained residue was dissolved in dichloromethane (20 mL), 2-bromothiazole-4-carboxylic acid (1.66 g, 8 mmol), 1-ethyl-3-(3-dimethylaminopropyl)carbodiimide (1.68 g, 8.8 mmol, 1.1 eq), hydroxybenzotriazole (1.19 g, 8.8 mmol, 1.1 eq), and diisopropylethylamine (4.2 mL, 24 mmol, 3 eq) were added dropwise, and then stirring was performed at room temperature for 12 hours. Dilution with dichloromethane and then washing with water were performed. The organic solvent was extracted, dehydration with anhydrous sodium sulfate was performed, and separation was performed using column chromatography with a 20%-50% ethyl acetate/hexane solution to obtain yellow solid Compound a-2 (3.00 g, 63%).

¹H NMR (600 MHz, CDCl₃) δ 8.08 (s, 1H), 8.01 (d, J = 8.4 Hz, 1H), 7.69 - 7.65 (m, 4H), 7.48 - 7.44 (m, 2H), 7.42 - 7.37 (m, 4H), 6.92 - 6.90 (m, 1H), 4.44 (qd, J = 6.5, 1.9 Hz, 1H), 4.39 (dd, J= 8.4, 1.9 Hz, 1H), 4.02 - 3.97 (m, 1H), 3.55 (brs, 1H), 3.29 (app.t, J = 5.3 Hz, 2H), 1.22 (d, J = 6.5 Hz, 3H), 1.05 (s, 9H), 1.03 (d, J = 6.5 Hz, 3H); LCMS : 604.1 (M+H⁺) for C₂₇H₃₅BrN₃O₄SSi.

### Step 3: Preparation of Compound a-3

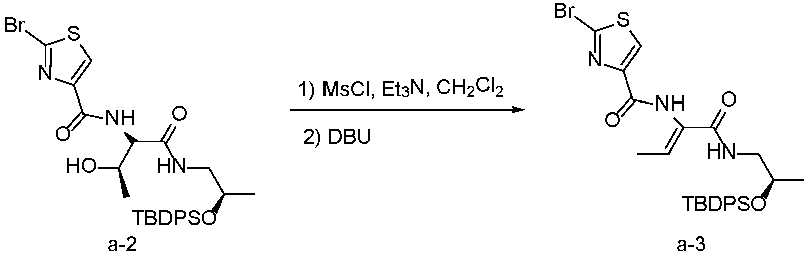

Compound a-2 (3 g, 4.96 mmol) was dissolved in dichloromethane (40 mL), methanesulfonyl chloride (MsCl, 1.15 mL, 14.9 mmol, 3 eq) and triethylamine (2.1 mL, 14.9 mmol, 3 eq) were added dropwise, and stirring was performed at room temperature for 1 hour. Then, 1,8-diazabicyclo[5,4,0]undec-7-ene (DBU, 11.1 ml, 74.4 mmol, 15 eq) was added and stirring was performed at room temperature for 2 hours. After confirming reaction completion, dilution with dichloromethane was performed, and supersaturated ammonium chloride was added to wash. The organic solvent was extracted, dehydration with anhydrous sodium sulfate was performed, and separation was performed using column chromatography with a 30%-50% ethyl acetate/hexane solution to obtain white solid Compound a-3 (2.2 g, 75%).

¹H NMR (600 MHz, CDCl₃): δ 8.32 (s, 1H), 8.12 (s, 1H), 7.66 - 7.64 (m, 4H), 7.48 - 7.41 (m, 2H), 7.40 - 7.37 (m, 4H), 6.53 (q, *J* = 7.1 Hz, 1H), 6.39 (app.t, *J* = 5.3 Hz, 1H), 4.05 - 4.01 (m, 1H), 3.41 (ddd, J = 13.6, 5.4, 3.6 Hz, 1H), 3.33 (dt, *J* = 13.6, 5.9 Hz, 1H), 1.79 (d, *J* = 7.1, 3H), 1.10 (d, J = 6.2 Hz, 3H), 0.99 (s, 9H); LCMS : 586.1 (M+H⁺) for C₂₇H₃₃BrN₃O₃SSi.

### Step 4: Preparation of Compound a-4

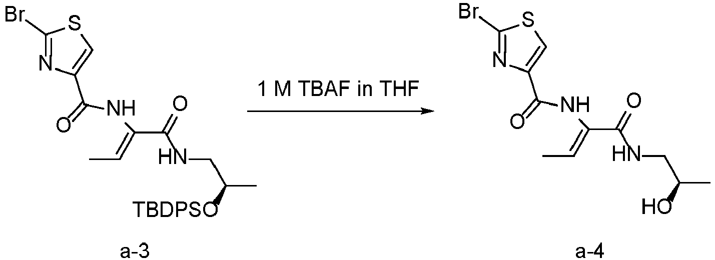

Compound a-3 (2.2 g, 3.74 mmol) was dissolved in tetrahydrofuran (30 mL), 1 M tetra-n-butylammonium fluoride (TBAF, 6.74 mL, 6.74 mmol, 1.8 eq) was added dropwise, and then stirring was performed at room temperature for 30 minutes. Dilution with ethyl acetate was performed, and supersaturated ammonium chloride was added to wash. The organic solvent was extracted, dehydration with anhydrous sodium sulfate was performed, and separation was performed using column chromatography with a (2%-5% methanol : 98%-95% dichloromethane) solution to obtain colorless liquid Compound a-4 (800 mg, 61%).

¹H NMR (600 MHz, CD₃OD): δ 8.30 (d, J = 0.9 Hz, 1H), 6.71 (q, J = 7.1 Hz, 1H), 3.94 - 3.85 (m, 1H), 3.30 (dd, J = 13.5, 4.7 Hz, 1H), 3.20 (dd, J = 13.4, 7.1 Hz, 1H), 1.79 (d, J = 7.1 Hz, 3H), 1.16 (d, J = 6.3 Hz, 3H); LCMS : 348.0 (M+H⁺) for C₁₁H₁₅BrN₃O₃S.

### Step 5: Preparation of Compound A1

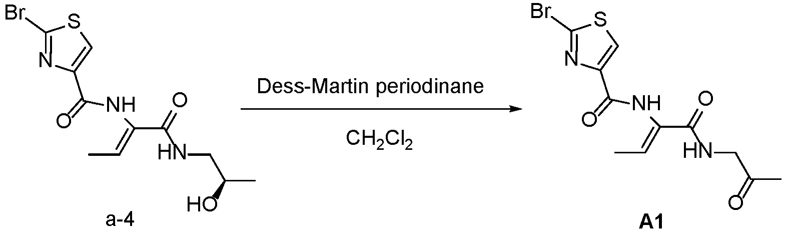

Compound a-4 (386 mg, 1.12 mmol) was dissolved in dichloromethane (10 mL), and then Dess-Martin periodinane (706 mg, 1.66 mmol, 1.5 eq) was added dropwise. Stirring was performed at room temperature for 2 hours, dilution with dichloromethane was performed, and washing with supersaturated sodium hydrogen carbonate was performed. The organic solvent was extracted, dehydration with anhydrous sodium sulfate was performed, and separation was performed using column chromatography with a (2%-5% methanol : 98%-95% dichloromethane) solution to obtain white solid Compound A1 (356 mg, 89%).

¹H NMR (600 MHz, CD₃OD): δ 8.30 (s, 1H), 6.77 (q, J = 7.1 Hz, 1H), 4.07 (s, 2H), 2.18 (s, 3H), 1.81 (d, J = 7.1 Hz, 3H); LCMS : 345.9 (M+H⁺) for C₁₁H₁₃BrN₃O₃S.

### [Example 1] Preparation of Compounds 13 (Micrococcin P2)

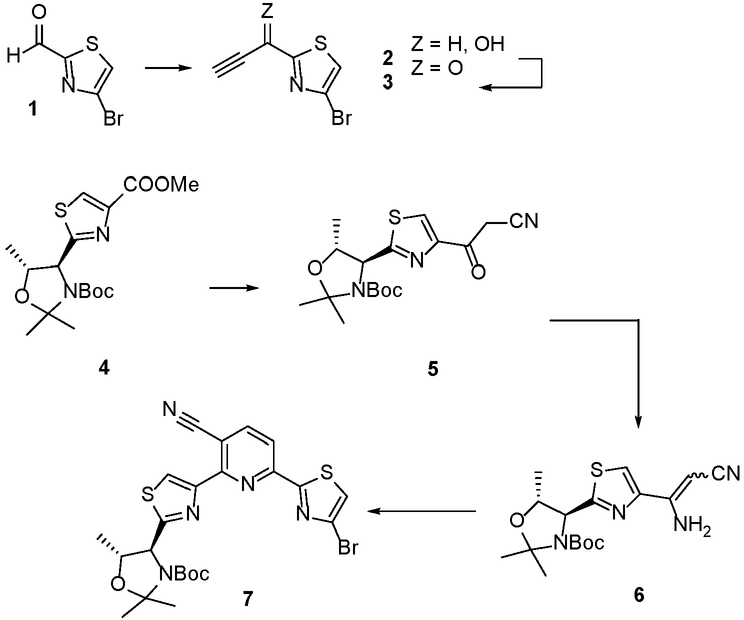

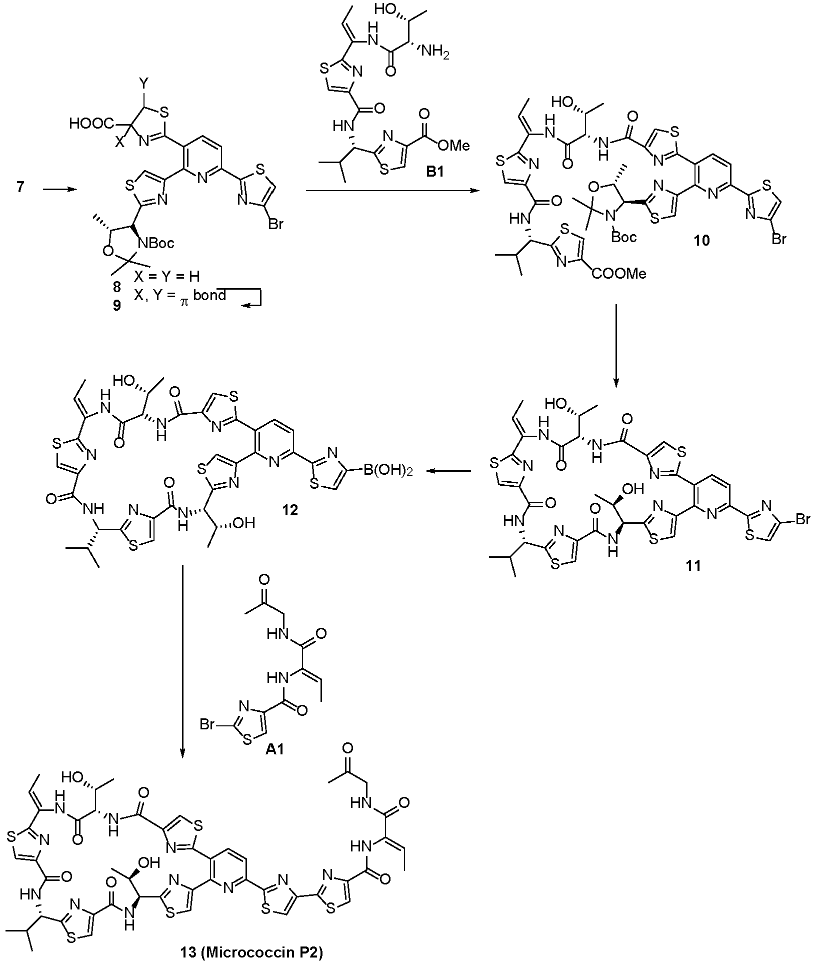

### Step 1: Preparation of Compound 3

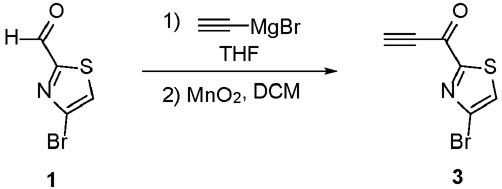

4-Bromo-2-formylthiazole (768 mg, 4 mmol) was dissolved in tetrahydrofuran (10 mL), and an ethynyl magnesium bromide solution (0.5 M in THF, 16 mL, 8 mmol) was slowly added dropwise at 0°C. Stirring was performed at room temperature for 30 minutes, and a supersaturated ammonium chloride solution was added to stop the reaction. Extraction with ethyl acetate, then dehydration with anhydrous sodium sulfate, and concentration under reduced pressure were performed. The obtained residue was separated using column chromatography with a 30% ethyl acetate/70% hexane solution to obtain a white solid compound. The obtained compound (632 mg, 2.9 mmol) was dissolved in dichloromethane (30 mL), manganese dioxide (2.52 g, 29 mmol) was added, and then stirring was performed at room temperature for 2 hours. A manganese residue was filtered using celite, extraction with dichloromethane and dehydration with anhydrous sodium sulfate were performed, and then concentration under reduced pressure was performed to obtain brown solid Compound **3** (527 mg, 61%).

¹H NMR (400 MHz, CDCl₃) δ 7.69 (s, 1H), 3.67 (s, 1H); LCMS : 215.9 (M+H⁺) for C₆H₃BrNOS.

### Step 2: Preparation of Compound 5

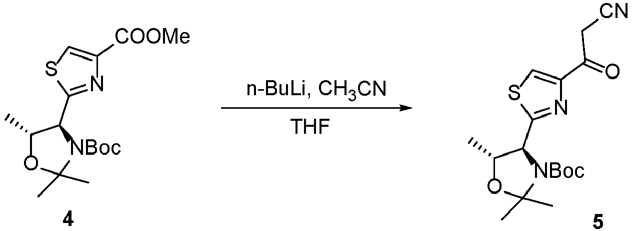

Acetonitrile (6.52 mL, 125 mmol, 2 eq) was dissolved in tetrahydrofuran (120 mL), and then a n-butyllithium solution (2.5 M in hexanes, 54.8 mL, 137 mmol, 2.2 eq) was slowly added dropwise at -78°C. Stirring was performed at -78°C for 20 minutes, and then Compound **4** (22.3 g, 62.6 mmol) synthesized by the method disclosed in Org. Lett, 2020, 22, 6, 2365-2370, which was dissolved in tetrahydrofuran (120 mL), was slowly added dropwise at the same temperature. The temperature was slowly raised to room temperature, the reaction was performed for 1 hour, and hydrochloric acid at a concentration of 1 M was added to stop the reaction. Dilution with ethyl acetate was performed, and hydrochloric acid was further added to perform acidification to pH 3. The organic solvent was extracted, dehydration with anhydrous sodium sulfate was performed, and concentration under reduced pressure was performed to obtain brown solid Compound **5** (22.2 g, 97%).

¹H NMR (400 MHz, CDCl₃) δ 8.29 (s, 1H), 4.71 (d, J = 17.7 Hz, 1H), 4.32 - 4.09 (m, 3H), 1.70 (broad peak, 6H), 1.54 - 1.38 (m, 7H), 1.31 - 1.06 (m, 5H); LCMS : 366.1 (M+H⁺) for C₁₇H₂₄N₃O₄S.

### Step 3: Preparation of Compound 6

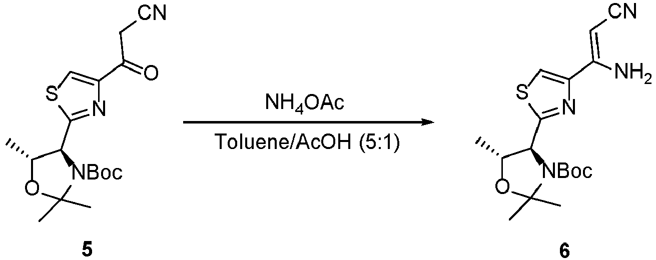

Compound **5** (22.2 g, 61.0 mmol) was dissolved in a mixture of toluene (300 mL) and acetic acid (60 mL), and ammonium acetate (47 g, 610 mmol, 10 eq) was added dropwise. Stirring was performed at 120°C for 1 hour, and slow cooling to room temperature was performed. Dilution with ethyl acetate, and then washing with a supersaturated sodium hydrogen carbonate solution and water were performed. The organic solvent was extracted, dehydration with anhydrous sodium sulfate was performed, and concentration under reduced pressure was performed to obtain yellow oil Compound **6** (21.0 g, 94%).

¹H NMR (400 MHz, CDCl₃) δ 7.60 (s, 1H), 5.59 - 5.51 (m, 2H), 4.81 - 4.50 (m, 2H), 4.21 - 4.10 (m, 1H), 1.67 (s, 6H), 1.48 - 1.36 (m, 6H), 1.27 - 0.96 (m, 6H); LCMS : 365.1 (M+H⁺) for C₁₇H₂₅N₄O₃S.

### Step 4: Preparation of Compound 7

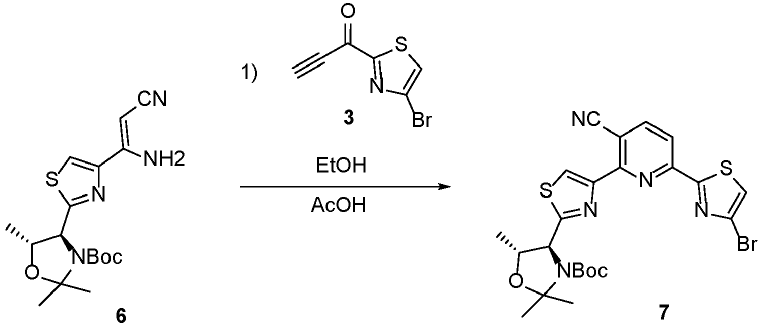

Compound **6** (21.0 g, 57.5 mmol) was dissolved in ethanol (300 ml), Compound **3** (22.9 g, 106.3 mmol, 1.85 eq) was added dropwise, and stirring was performed at 60°C for 90 minutes. After confirming that the starting material disappeared with TLC and LC mass, acetic acid (30% volume, 90 ml) was added, and then the reaction was performed at 90°C for 16 hours. Concentration under reduced pressure was performed to remove ethanol and acetic acid, dilution with dichloromethane was performed, and the reaction was stopped with a supersaturated sodium hydrocarbon carbonate solution. The organic solvent was extracted, dehydration with anhydrous sodium sulfate was performed, and concentration under reduced pressure was performed. Separation using column chromatography with a 5%-15% ethyl acetate/hexane solution was performed to obtain yellow solid Compound **7** (16.5 g, 51%).

¹H NMR (400 MHz, CDCl₃) δ 8.27 (s, 1H), 8.19 (s, 2H), 7.48 (s, 1H), 4.85 (d, *J* = 7.3 Hz, 1H), 4.58 - 4.23 (broad peak, 1H), 1.83 - 1.67 (broad peak, 6H), 1.57 - 1.45 (m, 6H), 1.33 - 1.20 (m, 6H); LCMS : 562.0 (M+H⁺) for C₂₃H₂₅BrN₅O₃S₂.

### Step 5: Preparation of Compound 9

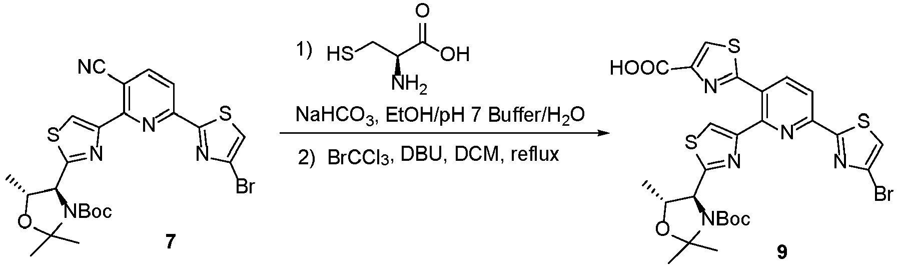

Compound **7** (1.42 g, 2.53 mmol) was dissolved in a mixed solution of ethanol (27 mL), a sodium phosphate buffer solution (pH 7, 9 mL), and water (4.5 mL), and then L-cysteine (3.06 g, 25.3 mmol, 10 eq) was added dropwise. Stirring was performed at 100°C for 24 hours, concentration under reduced pressure was performed to remove ethanol, and dilution with ethyl acetate was performed. Washing with hydrochloric acid at a concentration of 1 M was performed for removing an excessive amount of cysteine, and dehydration was performed with anhydrous sodium sulfate. The solution obtained by extraction was concentrated under reduced pressure to obtain thiazoline of brown oil.

The thiazoline of brown oil (1.68 g, 2.53 mmol) was dissolved in dichloromethane (30 mL), bromotrichloromethane (1.0 mL, 10.12 mmol, 4 eq) was added, and then 1,8-diazabicyclo[5,4,0]undec-7-ene (1.88 mL, 12.65 mmol, 5 eq) was slowly added dropwise. Stirring was performed at 60°C for 4 hours, and hydrochloric acid at a concentration of 1 M was added to stop the reaction. Extraction with dichloromethane was performed, and then washing with water and brine was performed. The extracted organic solvent was dehydrated with anhydrous sodium sulfate, concentrated under reduced pressure, and separated using column chromatography with a 5%-15% methanol/dichloromethane solution to obtain brown solid Compound **9** (1.05 g, 62%).

¹H NMR (400 MHz, CDCl₃) δ 8.35 - 8.05 (m, 3H), 7.89 (bs, 1H), 7.33 (s, 1H), 4.49 - 4.40 (m, 1H), 4.09 - 3.97 (m, 1H), 1.71 - 1.29 (m, 10H), 1.26 - 1.08 (m, 8H); LCMS : 664.0 (M+H⁺) for C₂₆H₂₇BrN₅O₅S₃.

### Step 6: Preparation of Compound 10

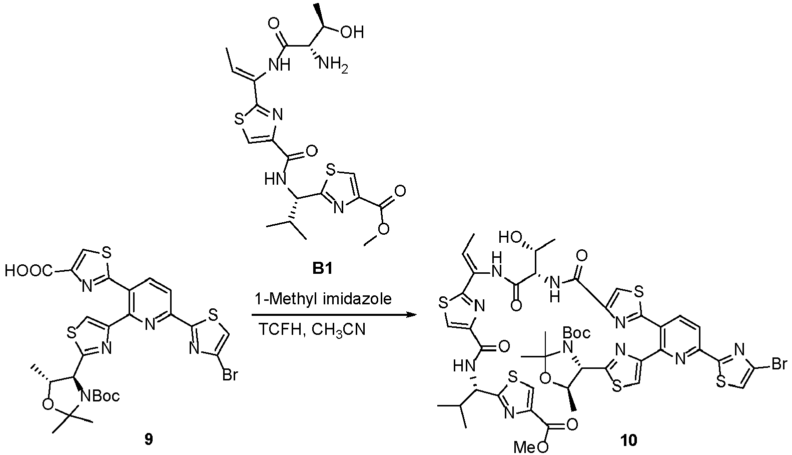

Compound **B1** was prepared according to Org. Lett, 2020, 22, 6, 2365-2370. Compound **9** (1.05 g, 1.57 mmol) was dissolved in acetonitrile (8 mL), Compound **B1** (932 mg, 1.88 mmol), 1-methylimidazole (0.376 mL, 4.71 mmol, 3 eq), and chloro-*N,N,N,N*-tetramethylformamidinium hexafluorophosphate (TCFH, 0.528 g, 1.88 mmol, 1.2 eq) were added, and stirring was performed at room temperature for 30 minutes. The solution was diluted with ethyl acetate and washed with water, an organic layer was dehydrated using anhydrous sodium sulfate, and concentration under reduced pressure was performed. The concentrated solution was separated using column chromatography with a (40%-90 % ethyl acetate: 60%-10% hexane) solution to obtain white solid Compound **10** (1.05 g, 60%).

¹H NMR (400 MHz, CDCl₃) δ 8.69 (s, 1H), 8.44 (d, *J* = 9.5 Hz, 1H), 8.35 - 8.24 (m, 4H), 8.24 - 8.17 (m, 1H), 8.06 (s, 1H), 7.96 (s, 1H), 7.94 - 7.87 (m, 1H), 6.47 (q, 7.1 Hz, 1H), 5.41 - 5.31 (m, 1H), 4.88 (d, *J* = 5.3 Hz, 1H), 4.78 (bs, 1H), 4.68 - 4.58 (m, 1H), 4.58 - 4.46 (m, 1H), 4.16 - 3.98 (m, 1H), 3.86 (s, 3H), 2.49 - 2.45 (m, 1H), 1.87 (d, *J* = 7.1 Hz, 3H), 1.69 - 1.48 (m, 6H), 1.45 - 1.32 (m,3H), 1.31 - 1.08 (m, 12H), 1.03 (d, *J* = 6.8 Hz, 3H), 0.95 (d, *J* = 6.7 Hz, 3H) ; LCMS : 1127.1 (M+H⁺) for C₄₆H₅₂BrN₁₀O₉S₅.

### Step 7: Preparation of Compound 11

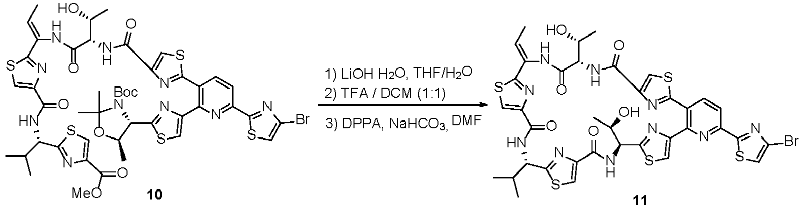

Compound **10** (200 mg, 0.177 mmol) was added to a mixed solvent of tetrahydrofuran (1 mL)/ water (1 mL), lithium hydroxide monohydrate (22 mg, 0.531 mmol, 3 eq) was added, and stirring was performed at room temperature for 2 hours. The solution was diluted with a dichloromethane solution, washed with a 1 M hydrochloric acid solution, dehydrated using anhydrous sodium sulfate, and concentrated under reduced pressure to obtain carboxylic acid. The concentrated solution was added to a mixed solvent of dichloromethane (1 mL)/ trifluoroacetic acid (1 mL), stirring was performed at room temperature for 30 minutes, and then concentration under reduced pressure was performed to obtain a trifluoroacetic acid salt. The concentrated solution was diluted with DMF (1 mL), diphenylphosphoryl azide (DPPA, 0.038 mL, 0.177 mmol, 1 eq) and sodium hydrogen carbonate (0.148 g, 1.77 mmol, 10 eq) were added, and stirring was performed at room temperature for 12 hours. The solution was diluted with a dichloromethane solution, an organic layer was washed with water three times, dehydrated with anhydrous sodium sulfate, concentrated under reduced pressure, and separated using column chromatography with a (2%-5% methanol: 98%-95% dichloromethane) solution to synthesize yellow solid Compound **11** (96 mg, 56%).

¹H NMR (400 MHz, CDCl₃) δ 8.77 (s, 1H), 8.39 (d, *J* = 9.6 Hz, 1H), 8.22 - 8.18 (m, 2H), 8.16 - 8.09 (m, 3H), 8.01 - 7.92 (m, 3H), 7.40 (s, 1H), 6.39 (q, *J* = 7.0 Hz, 1H), 5.27 - 5.18 (m, 2H), 4.92 (d, *J* = 7.8 Hz, 1H), 4.67 (s, 1H), 4.47 - 4.33 (m, 1H), 2.95 (bs, 1H), 2.48 (dq, *J* = 13.4, 6.6 Hz, 1H), 2.22 (bs, 1H), 1.81 (d, *J* = 7.0 Hz, 3H), 1.57 (d, *J* = 5.2 Hz, 3H), 1.19 (d, *J* = 6.5 Hz, 3H), 1.14 (d, *J* = 6.7 Hz, 3H), 0.95 (d, *J* = 6.6 Hz, 3H) ; LCMS : 955.0 (M+H⁺) for C₃₇H₃₆BrN₁₀O₆S₅.

### Step 8: Preparation of Compound 12

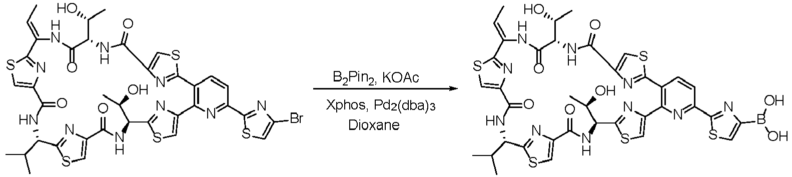

Compound **11** (41 mg, 0.043 mmol) was dissolved in 1,4-dioxane (0.6 mL), and bis(pinacolato)diborone (B₂Pin₂, 14.2 mg, 0.056 mmol, 1.3 eq), potassium acetate (KOAc, 6.7 mg, 0.065 mmol, 1.5 eq), and Xphos (3.1 mg, 15 mol%) were added. After the addition, nitrogen was added for 1 minute, tris(dibenzylideneacetone)dipalladium(0) (Pd₂(dba)₃, 4mg, 10 mol%) was added dropwise. Stirring was performed at 90°C for 3 hours, dilution with dichloromethane was performed, and the organic solvent was extracted. The extracted organic solvent was washed with water, dehydrated with anhydrous sodium sulfate, and concentrated under reduced pressure to obtain solid Compound **12** (18 mg, 45%).

¹H NMR (400 MHz, MeOD) δ 8.40 - 8.21 (m, 4H), 8.13 (s, 1H), 7.93 (s, 1H), 7.76 (s, 1H), 6.61 (q, *J* = 6.9 Hz, 1H), 5.29 - 5.19 (m, 2H), 4.75 (d, *J* = 3.1 Hz, 1H), 4.62 - 4.50 (m, 1H), 4.30 (dd, *J* = 6.3, 2.9 Hz, 1H), 2.64 (dq, *J* = 13.4, 6.7 Hz, 1H), 1.85 (d, *J* = 7.0 Hz, 3H), 1.53 (d, *J* = 6.4 Hz, 3H), 1.19 (d, *J* = 6.3 Hz, 3H), 1.13 (d, *J* = 6.7 Hz, 3H), 0.99 (d, *J* = 6.7 Hz, 3H) ; LCMS : 921.1 (M+H⁺) for C₃₇H₃₈BN₁₀O₈S₅.

### Step 9: Preparation of Compound 13

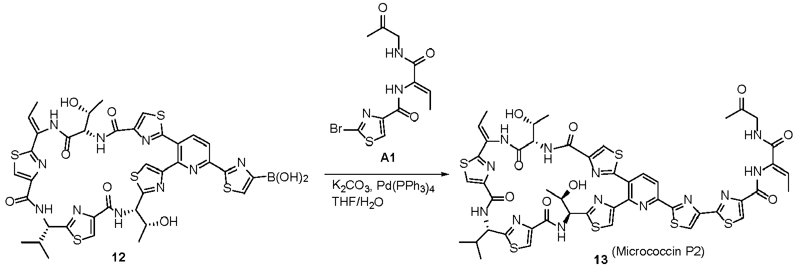

Compound **12** (276 mg, 0.3 mmol) was dissolved in a mixture of tetrahydrofuran (6 mL) and water (1.5 mL). Compound **A1** (155 mg, 0.45 mmol, 1.5 eq) was added dropwise, and nitrogen was supplied for 3 minutes. Thereafter, potassium carbonate (124 mg, 0.9 mmol, 3 eq) and tetrakis(triphenylphosphine) palladium (Pd(PPh₃)₄, 10 mol%, 35 mg) were added, and nitrogen was added again for 1 minute. Stirring was performed at 38°C for 3 hours, and then concentration under reduced pressure was performed. The obtained residue was separated using column chromatography with a 0-5% methanol/dichloromethane solution to obtain white solid Compound **13** (179 mg, 55%).

¹H NMR (600 MHz, MeOD) δ 8.40 - 8.31 (m, 4H), 8.27 (s, 1H), 8.22 (s, 1H), 8.13 (s, 1H), 7.96 (s, 1H), 6.82 (q, *J* = 7.1 Hz, 1H), 6.60 (q, *J* = 6.8 Hz, 1H), 5.24 (d, *J* = 2.9 Hz, 1H), 5.22 (d, *J* = 8.8 Hz, 1H), 4.78 (d, *J* = 3.1 Hz, 1H), 4.62 - 4.55 (m, 1H), 4.30 (qd, *J* = 6.3, 2.8 Hz, 1H), 4.10 (d, *J* = 13.0 Hz, 1H), 2.63 (dq, *J* = 13.4, 6.7 Hz, 1H), 2.19 (s, 3H), 2.20 - 2.14 (m, 1H), 1.92 (s, 1H) 1.86 (app. d, *J* = 7.0 Hz, 6H), 1.55 (d, *J* = 6.4 Hz, 3H), 1.18 (d, J = 6.3 Hz, 3H), 1.13 (d, *J* = 6.7 Hz, 3H), 0.99 (d, *J* = 6.7 Hz, 3H); LCMS : 1142.2 (M+H⁺) for C₄₈H₄₈N₁₃O₉S₆.

### [Example 2] Antibacterial activity experiment against tuberculosis bacteria (M. tuberculosis)

The micrococcin compound (Compound **13;** Micrococcin P2) prepared in Example 1 of the present invention was used to measure antibacterial activity.

A liquid medium for tuberculosis bacteria culture was obtained by adding 10% OADC (oleic acid, albumin, dextrose, catalase; Becton Dickinson) and 0.05% Tween 80 (Sigma Aldrich) to a Middlebrook 7H9 medium (Becton Dickinson), and a solid medium was obtained by adding 10% OADC to a Middlebrook 7H10 medium (Becton Dickinson). Drug-resistant strains including standard tuberculosis strains were cultured in the Middlebrook 7H9 liquid medium, dilution was performed to be 4.0×10⁵ cell/well, and the solution was dispensed in a 96-well plate. The compounds were treated to be DMSO 1%, a 0.025% resazurin (REMA) solution was dispensed after 5 days, culture was performed for 24 hours, and growth of bacteria was confirmed. The strains used in the experiment are shown in the following Table 1. As the antibacterial activity, a MIC₅₀ value which is an antibiotic concentration to inhibit growth of bacteria up to 50% as compared with growth of control was measured by resazurin (excitation: 530nm, emission: 590nm), and the MIC₅₀ value was calculated by Prims 6. The results are shown in the following Table 2.

The antibiotic resistance of the strains used in tuberculosis evaluation in the following Table 1 is as follows:
INH: Isoniazid, RIF: Rifampicin, LEV: Levofloxacin, OFX: Ofloxacin, MXF: Moxifloxacin, KM: Kanamycin, AMK: Amikacin, CPM: Capreomycin

**[Table 1]**

| | Strain # | INH | RIF | LEV | OFX | MXF | KM | AMK | CPM |
|---|---|---|---|---|---|---|---|---|---|
| *M*. *tuberculosis* | H37Rv | S | S | S | S | S | S | S | S |
| Pan-Susceptible | Isolate #2 | S | S | S | S | S | S | S | S |
| | Isolate #4 | S | S | S | S | S | S | S | S |
| | Isolate #6 | S | S | S | S | S | S | S | S |
| | Isolate #7 | S | S | S | S | S | S | S | S |
| Multi Drug Resistant (MDR) | Isolate #3 | R | R | S | S | S | S | S | S |
| | Isolate #4 | R | R | S | S | S | S | S | S |
| | Isolate #11 | R | R | S | S | S | S | S | R |
| | Isolate #20 | R | R | S | S | R | S | S | R |
| | Isolate #21 | R | R | S | S | S | R | R | R |
| | Isolate #30 | R | R | R | R | R | S | S | R |
| Extensively Drug Resistant (XDR) | XDR-3 | R | R | R | R | R | R | R | R |
| | XDR-4 | R | R | R | R | R | R | R | R |
| | XDR-5 | R | R | R | R | R | R | R | R |
| | XDR-6 | R | R | R | R | R | R | R | R |
| | XDR-10 | R | R | R | R | R | R | R | R |

| | | | | | | | | | |
|---|---|---|---|---|---|---|---|---|---|
| [236] S: susceptible, R: resistant | | | | | | | | | |

**[Table 2]**

| | Strain # | Micrococcin P2 (MIC₅₀) |
|---|---|---|
| *M. tuberculosis* H37Rv | H37Rv | 89 nM |
| Pan-susceptible isolate #2 | Pan-S2 | 199 nM |
| Pan-susceptible isolate #4 | Pan-S4 | 356 nM |
| Pan-susceptible isolate #6 | Pan-S6 | 354 nM |
| Pan-susceptible isolate #7 | Pan-S7 | 208 nM |
| Multi Drug Resistant isolate #3 | MDR-3 | 73 nM |
| Multi Drug Resistant isolate #4 | MDR-4 | 169 nM |
| Multi Drug Resistant isolate #11 | MDR-11 | 51 nM |
| Multi Drug Resistant isolate #20 | MDR-20 | 2.3 µM |
| Multi Drug Resistant isolate #21 | MDR-21 | 44 nM |
| Multi Drug Resistant isolate #30 | MDR-30 | 304 nM |
| Extensively Drug Resistant #3 | XDR-3 | 375 nM |
| Extensively Drug Resistant #4 | XDR-4 | 94 nM |
| Extensively Drug Resistant #5 | XDR-5 | 114 nM |
| Extensively Drug Resistant #6 | XDR-6 | 722 nM |
| Extensively Drug Resistant #10 | XDR-7 | 209 nM |

As seen in Table 2, it is shown that the micrococcin compound of the present invention had a very improved antibacterial effect against multidrug-resistant or extensive drug-resistant tuberculosis as well as susceptible tuberculosis, and thus, may be very useful as a therapeutic agent of multidrug-resistant or extensive drug-resistant tuberculosis.

### [Example 3] Antibacterial activity experiment against nontuberculous mycobacteria (NTM)

The micrococcin compound (Compound **13;** Micrococcin P2) prepared in Example 1 of the present invention was used to measure antibacterial activity against nontuberculous mycobacteria. The strains selected for evaluating efficacy of Micrococcin P2 against nontuberculous mycobacteria were *M. avium* ATCC 700898, *M. intracellulare* ATCC 13950, and *M. abscessus* ATCC 19977.

A Mycobacterium culture medium which was cultured until a late log phase was diluted to be 4.0×10⁵ cell/well in a Middlebrook 7H10 agar medium (Difco) supplemented with 10% OADC (oleic acid, albumin, dextrose, catalase; Becton Dickinson) and 0.05% Tween 80 (Sigma Aldrich) for an antibacterial activity test, and dispensed in a 96-well plate. The compounds were treated to be DMSO 1%, a 0.025% resazurin (REMA) solution was dispensed after 3 days and cultured, and growth of bacteria was confirmed. As the antibacterial activity, a MIC₅₀ value which is an antibiotic concentration to inhibit growth of bacteria up to 50% as compared with growth of control was measured by resazurin (excitation: 530nm, emission: 590nm). The MIC₅₀ value was calculated by Prism 6 and the results are shown in the following Table 3.

As a control drug, clarithromycin and rifampicin which are used in NTM treatment were used, and all drugs were used in stepwise concentration gradients of 11 steps from 5 µM.

**[Table 3]**

| Non-tuberculosis Mycobacterium Species | Strain | MIC₅₀ | | |
|---|---|---|---|---|
| | | Micrococcin P2 | CLR | RIF |
| *Mycobacterium avium* | ATCC 700898 | 28 nM | 76 nM | 6.6 µM |
| *Mycobacterium intracellulare* | ATCC 13950 | 33 nM | 143 nM | 380 nM |
| *Mycobacterium abscessus* | ATCC 19977 | 654 nM | - | - |

| | | | | |
|---|---|---|---|---|
| CLR: Clarithromycin, RIF: Rifampicin | | | | |

As seen in Table 3, it is shown that the micrococcin compound of the present invention had an improved antibacterial effect as compared with clarithromycin and rifampicin which are used in the NTM treatment. In particular, it is shown to be effective against *Mycobacterium avium and Mycobacterium intracellulare.*

### [Example 4] Antibacterial activity experiment against Clostridium difficile

Inhibitory activity against C. *difficile* was measured through clinical strains distributed from Korea Culture Collection of Antimicrobial Resistance Microbes or strains distributed from American Type Culture Collection (ATCC). The inhibitory activity of MIC was evaluated by an agar medium dilution method according to the Clinical and Laboratory Standards Institute guidelines (CLSI) [Methods for Antimicrobial Susceptibility Testing of Anaerobic Bacteria; Approved Standard-Seventh Edition, CLSI document M11-A7, 2007].

*C*. In a liquid culture of *C*. *difficile,* a BHIS medium (Brain heart infusion-supplemented medium) in which a 0.5% yeast extract (5g/1L, Becton Dickinson) was added to a BHI medium (Brain heart infusion, Becton Dickinson) and a supplemented Brucella agar (Becton Dickinson) as an agar medium were used. All tested compounds were made into an agar medium to have a concentration of 32 ug/ml to 0.06 ug/ml. A day before culturing *C*. *difficile* strains in a liquid, a BHIS medium was added to an anaerobic chamber and oxygen in the liquid medium (broth) was removed for a day. A day before the experiment, bacteria were cultured in 4 ml of the BHIS medium. Bacteria cultured for a day was diluted to be about 4-5×10⁷ CFU/ml using the BHIS medium. The diluted bacterial fluid was added dropwise at 10 µl each time to a medium containing an antibiotic and cultured in the anaerobic chamber for 18 hours or more, and the results were observed and are shown in the following Table 4:

**[Table 4]**

| | | MIC (µg/ml) | | | |
|---|---|---|---|---|---|
| Organism | Strain/No. | Micrococcin P2 | VAN | MDZ | MXF |
| *C. difficile* | ATCC 9689 | 0.5 | 2 | 1 | 1 |
| *C. difficile* | ATCC 17858 | 1 | 1 | 1 | 2 |
| *C. difficile* | ATCC 43255 | 2 | 1 | 2 | 2 |
| *C. difficile* | ATCC 51695 | 1 | 2 | 2 | 2 |
| *C. difficile* | ATCC BAA-1871 | 1 | 1 | 2 | 32 |
| *C. difficile* | Clinical isolate #1 | 1 | 4 | 1 | 32 |
| *C. difficile* | Clinical isolate #2 | 1 | 1 | 2 | 2 |

| | | | | | |
|---|---|---|---|---|---|
| VAN: Vancomycin, MDZ: Metronidazole, MXF: Moxifloxacin | | | | | |

As seen in Table 4, it is shown that the micrococcin compound of the present invention had significantly improved antibacterial activity at a significantly low concentration as compared with currently used vancomycin, metronidazole, and moxifloxacin.

### [Example 5] Evaluation of efficacy in clostridium difficile -infected model

In order to culture *C*. *difficile* bacteria, a mixture of a SMC medium (bactopeptone: 0.9% protease: 0.5%, NH₄SO₄: 0.1%, tris buffer 0.15%) and BHIS agar medium at a ratio of 7:3 was used.

A C57BL/6J male 4 week old mouse was purchased and went through an adaptation period of 1 week, and the 5-6 week old mouse of 20-21 g was used as an experimental animal for evaluating efficacy. In order to ensure that the mouse is easily infected by inducing imbalance of intestinal microorganisms in the mouse, the mouse was provided with drinking water containing 2% DSS 5 days before infection, and 20 mg/kg of clindamycin was intraperitoneally injected into the mouse one day before infection. That is, drinking water containing 2% DSS (dextran sodium sulfate, MP biomedical) was provided for 5 days before infection, and general water without DSS was provided after infection.

As C. *difficile* used in infection, ribotype 027 clinical isolated strains (C. *difficile* Ribotype 027) were used, the bacteria were cultured in an anaerobic chamber, inoculated in a stock 5 days before infection, cultured for 2 days, subcultured, and grown for a day. In order to form spores to be used in infection, the bacteria were streaked in about 60 BHIS 7:3 media and cultured for two days.

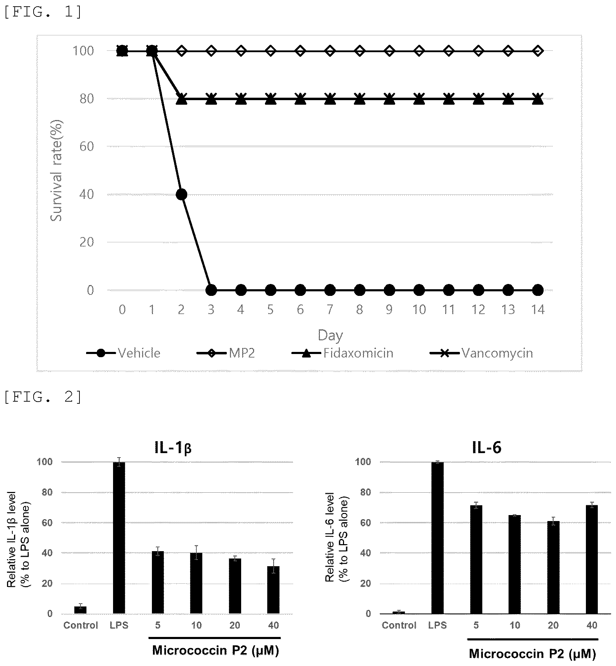

On the day of the experiment, the cultured bacterial fluid in a SMC:BHIS agar (7:3) medium was collected with a scraper and centrifuged at 6000 rpm for 15 minutes, a supernatant was discarded, and only a pellet was collected. 0.9% NaCl (saline) was added to the collected pellet (that is, bacterial body collected by centrifugation) to measure an optical density (OD) value, and an absorbance OD value dilution factor was 1/10 so that the 1/10 OD₆₀₀ value was 2.0. Thereafter, boiling in a water bath at 60°C was performed in order to remain only spores, and then a cooled bacterial liquid was orally administered to the mouse to induce colitis in the intestine. Thereafter, 30 mg/kg of vancomycin, micrococcin P2(MP2), and fidaxomicin compounds were orally administered once a day for 5 days, and the survival rate of the mouse was confirmed for about 2 weeks. The results are shown in FIG. 1.

As shown in FIG. 1, in the case of the micrococcin compound of the present invention and the control compounds, no death individual occurred by C. *difficile* recurrence even after drug administration.

In particular, the micrococcin compound of the present invention showed a survival rate of 100%, and showed excellent efficacy as compared with vancomycin and fidaxomicin which are currently used.

### [Example 6] Evaluation of anti-inflammatory efficacy

In order to confirm the anti-inflammatory effect of the micrococcin compound (Compound **13;** Micrococcin P2) prepared in Example 1 of the present invention, an inflammatory cytokine secretion amount was measured.

### 1) Cell culture

RAW 264.7 cells which are mouse-derived macrophage cell lines were distributed from Korean Cell Line Bank (KCLB), and a DMEM (Dulbeccon's modified Eagle's medium; PAA, Austria) medium including 1% Penicillin-Streptomycin and 10% FBS (fetal bovine serum; Gibco Laboratories) was used. Cells were cultured under the conditions of 37°C and 5% CO₂.

### 2) Measurement of inflammatory cytokine secretion amount

The cultured RAW 264.7 cells were dispensed at 2×10⁵ cells/well in a 24-well plate, the micrococcin compound (Compound **13;** Micrococcin P2) prepared in Example 1 was treated by concentrations (5, 10, 20, 40 µg/mL) and then treated 1 ug/mL of lipopolysaccharide (LPS), and culture was performed in an incubator (5% CO₂, 37°C) for 24 hours.

A control group was a cell group which was not treated, and as a negative control group, a cell group treated with only LPS was used.

After completion of culture, a medium of each well was collected (at 4°C, 12,000 rpm for 10 minutes) and centrifuged, a supernatant was taken, and the concentration of inflammatory cytokine (IL-6, Il-1β) in the medium was quantified using an ELISA kit (R&D system, BD). The results are shown in FIG. 2.

LPS increased secretion of inflammatory cytokines IL-6 (interleukin-6) and Il-1β (interleukin-1β), but when the treatment was performed with both LPS and the micrococcin compound of the present invention (Micrococcin P2), it was confirmed that the secretion amounts of inflammation-related cytokines IL-6 and Il-1β were significantly decreased.

It is shown therefrom that the micrococcin compound according to the present invention (Micrococcin P2) showed an anti-inflammatory effect through inflammatory cytokine inhibitory activity, and furthermore, may be used as pharmaceutical and functional food and cosmetic materials for treating, preventing, and improving inflammation-related diseases.

## Claims

1. A pharmaceutical composition for treating or preventing a bacterial infection caused by tuberculosis bacteria or nontuberculous mycobacteria comprising: a micrococcin compound represented by the following Chemical Formula 1, a solvate thereof, a hydrate thereof, a prodrug thereof, an isomer thereof, or a pharmaceutically acceptable salt thereof as an effective component:

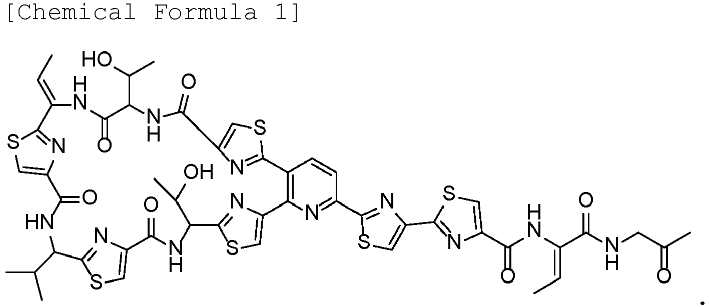

2. The pharmaceutical composition of claim 1, wherein the micrococcin compound is represented by the following Chemical Formula 2:

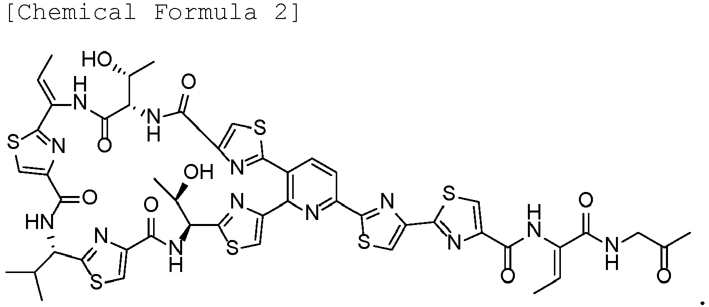

3. The pharmaceutical composition of claim 1, wherein the bacterial infection is tuberculosis or a symptom caused by the nontuberculous mycobacteria.

4. The pharmaceutical composition of claim 3, wherein the nontuberculous mycobacteria are selected from *Mycobacterium avium, Mycobacterium intracellulare, Mycobacterium abscessus, Mycobacterium kansasii, Mycobacterium fortuitum, Mycobacterium gordonae, Mycobacterium osloensis, Mycobacterium phlei, Mycobacterium smegmatis, Mycobacterium terrae, Mycobacterium chelonae, Mycobacterium mucogenicum, Mycobacterium peregrinum, Mycobacterium simiae, Mycobacterium wolinskyi,* or a combination thereof.

5. The pharmaceutical composition of claim 3, wherein the tuberculosis is susceptible tuberculosis, multidrug-resistant tuberculosis, or extensive drug-resistant tuberculosis.

6. The pharmaceutical composition of claim 1, further comprising an antibiotic.

7. The pharmaceutical composition of claim 6, wherein the antibiotic is isoniazid, rifampicin, ethambutol, SQ-109, pyrazinamide, streptomycin, kanamycin, capreomycin, ethionamide, prothionamide, enviomycin, para-aminosalicylic acid, cycloserine, amikacin, levofloxacin, moxifloxacin, Gatifloxacin, ofloxacin, terizidone, thionamide, ethionamide, protionamide, clofazimine, linezolid, amoxicillin, clavulanate, thioacetazone, imipenem, cilastatin, clarithromycin, bedaquiline, delamanid, lmipenem, cilastatin, meropenem, or a combination thereof.

8. A pharmaceutical composition for treating or preventing a bacterial infection caused by *clostridium* bacteria comprising: a micrococcin compound represented by the following Chemical Formula 1, a solvate thereof, a hydrate thereof, a prodrug thereof, an isomer thereof, or a pharmaceutically acceptable salt thereof as an effective component:

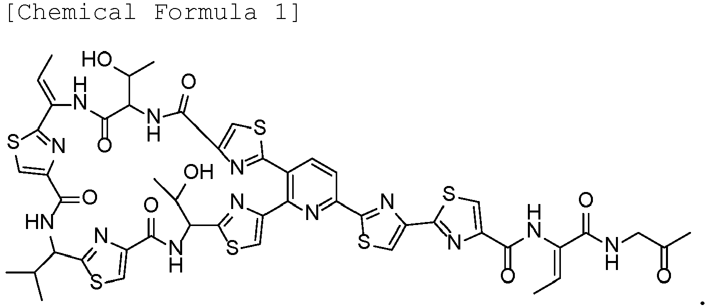

9. The pharmaceutical composition of claim 8, wherein the micrococcin compound is represented by the following Chemical Formula 2:

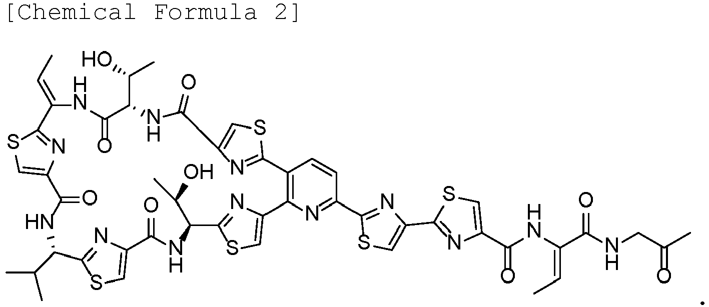

10. The pharmaceutical composition of claim 8, wherein the bacterial infection is selected from colitis, diarrhea, pseudomembranous colitis, gangrenous enteritis, infections of skin and soft tissue, food poisoning, or a combination thereof.

11. The pharmaceutical composition of claim 8, wherein the *clostridium* bacteria are selected from *Clostridium difficile, Clostridium perfringens, Clostridium cadaveris, Clostridium innocuum, Clostridium tetani, Clostridium bifermentans, Clostridium histolyticum, Clostridium clostridioforme, Clostridium subterminale, Clostridium ramosum, Clostridium septicum,* a combination thereof.

12. The pharmaceutical composition of claim 8, further comprising: one or two or more adjuvants selected from vancomycin, metronidazole, fidaxomicin, ridinilazole, actoxumab, bezlotoxumab, a probiotic, a prebiotic, intravenous immunoglubulin, and an antidiarrheal.

13. The pharmaceutical composition of claim 1 or 8, further comprising a pharmaceutically acceptable carrier.

14. The pharmaceutical composition of claim 1 or 8, wherein the micrococcin compound, the solvate thereof, the hydrate thereof, the prodrug thereof, the isomer thereof, or the pharmaceutically acceptable salt thereof are included at 0.001 to 10 wt% with respect to the total weight of the pharmaceutical composition.

15. A method for preparing a micrococcin compound, the method comprising:
subjecting a compound of the following Chemical Formula 3 to a boronization reaction to prepare a compound of the following Chemical Formula 4; and
subjecting the compound of the following Chemical Formula 4 to a coupling reaction with a compound of the following Chemical Formula 5 to prepare a micrococcin compound represented by the following Chemical Formula 1: 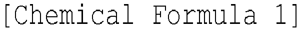 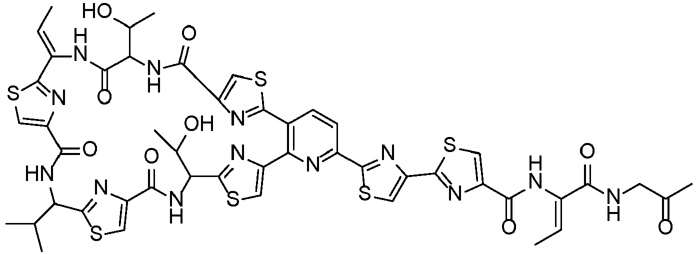 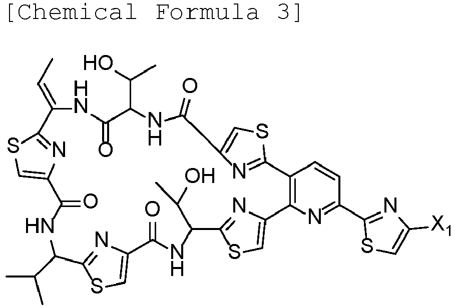 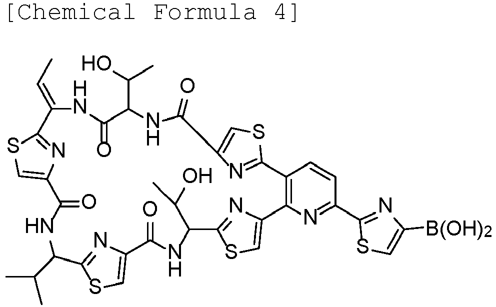 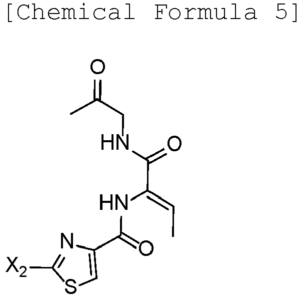
wherein
X₁ and X₂ are independently of each other a halogen.

16. The method for preparing a micrococcin compound of claim 15, wherein the compound of Chemical Formula 3 is prepared by including:
reacting a compound of the following Chemical Formula 3-1 with a compound of the following Chemical Formula 3-2 to prepare a compound of the following Chemical formula 3-3; and
subjecting the compound of Chemical Formula 3-3 to deprotection and intracyclic reactions to prepare the compound of Chemical Formula 3: 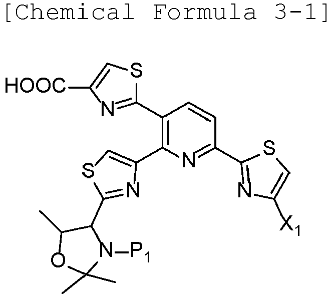 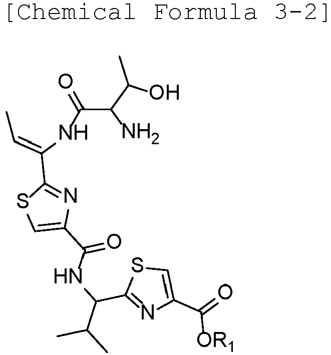 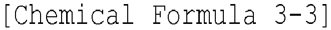 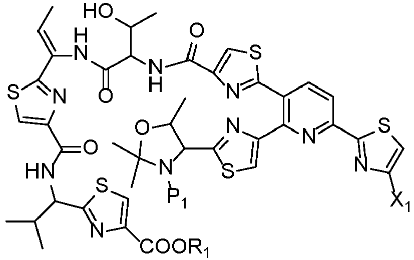
wherein
R₁ is independently of each other C1-C5 alkyl;
P₁ is a protection group; and
X₁ is a halogen.

17. The method for preparing a micrococcin compound of claim 16, wherein the compound of Chemical Formula 3-1 is prepared by including:
reacting a compound of the following Chemical Formula 2-1 and ammonium acetate to prepare a compound of the following Chemical Formula 2-2;
reacting the compound of Chemical Formula 2-2 with a compound of the following Chemical Formula 2-3 to prepare a compound of the following Chemical Formula 2-4; and
reacting the compound of Chemical Formula 2-4 with cysteine to prepare the compound of Chemical Formula 3-1: 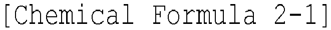 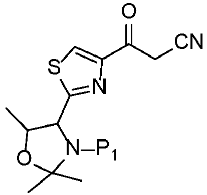 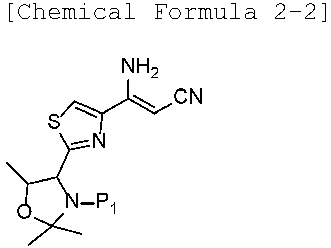 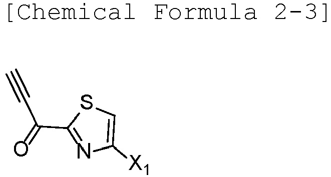 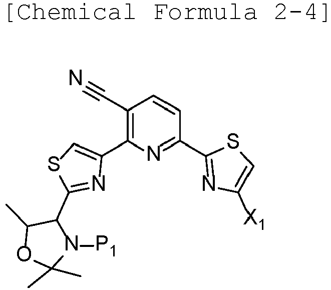
wherein
P₁ is a protection group; and
X₁ is a halogen.

18. An anti-inflammatory composition comprising: a micrococcin compound represented by the following Chemical Formula 1, a solvate thereof, a hydrate thereof, a prodrug thereof, an isomer thereof, or a pharmaceutically acceptable salt thereof as an effective component:

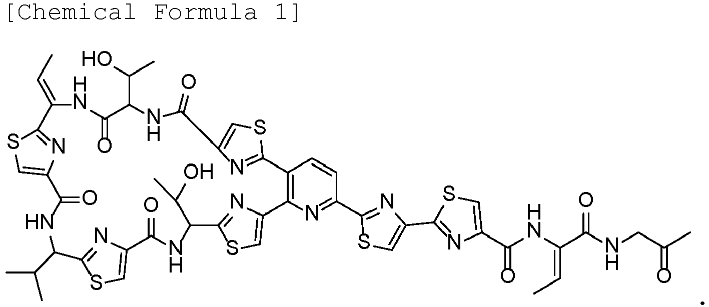

19. The anti-inflammatory composition of claim 18, wherein the micrococcin compound is represented by the following Chemical Formula 2:
